# EUROPÄISCHE PATENTSCHRIFT

(11) **EP 2 437 769 B1**
(45) Veröffentlichungstag und Bekanntmachung des Hinweises auf die Patenterteilung: **25.09.2019**
(21) Anmeldenummer: 10724472.5
(22) Anmeldetag: 02.06.2010
(51) Int. Cl.: A61K 38/16, C07K 14/47, C12N 15/62, C12N 15/74

(54) **REKOMBINANTE HERSTELLUNG VON PEPTIDEN**
RECOMBINANT PRODUCTION OF PEPTIDES
PRÉPARATION DE PEPTIDES DE MANIÈRE RECOMBINÉE

(30) Priorität: 03.06.2009 EP 09161837
(43) Veröffentlichungstag der Anmeldung: 11.04.2012
(73) Patentinhaber: BASF SE, 67056 Ludwigshafen am Rhein (DE)
(72) Erfinder: HÜMMERICH, Daniel, 67227 Frankenthal (DE); LIEBMANN, Burghard, 64625 Bensheim (DE); FEHR, Markus, 67346 Speyer (DE); SCHWALB, Carsten, 68199 Mannheim (DE); BRÜSER, Heike, 67346 Speyer (DE)
(74) Vertreter: BASF IP Association
(86) Internationale Anmeldenummer: PCT/EP2010/057726
(87) Internationale Veröffentlichungsnummer: WO 2010/139736

(56) Entgegenhaltungen:
- WO-A1-98/54336
- WO-A1-2007/025719
- WO-A1-2007/082936
- WO-A1-2008/155304
- WO-A1-2009/080306
- WO-A2-00/31279
- WO-A2-2006/008163
- LEE J H ET AL: "Acidic Peptide-Mediated Expression of the Antimicrobial Peptide Buforin II as Tandem Repeats inEscherichia coli", PROTEIN EXPRESSION AND PURIFICATION, ACADEMIC PRESS, SAN DIEGO, CA, Bd. 12, Nr. 1, 1. Februar 1998 (1998-02-01), Seiten 53-60, XP004447560, ISSN: 1046-5928, DOI: DOI:10.1006/PREP.1997.0814
- HA-KUN KIM ET AL: "Expression of the cationic antimicrobial peptide lactoferricin fused with the anionic peptide in Escherichia coli", APPLIED MICROBIOLOGY AND BIOTECHNOLOGY, SPRINGER, BERLIN, DE, Bd. 72, Nr. 2, 19. Januar 2006 (2006-01-19), Seiten 330-338, XP019421997, ISSN: 1432-0614, DOI: DOI:10.1007/S00253-005-0266-5
- METLITSKAIA LUBA ET AL: "Recombinant antimicrobial peptides efficiently produced using novel cloning and purification processes.", BIOTECHNOLOGY AND APPLIED BIOCHEMISTRY JUN 2004 LNKD- PUBMED:15154847, Bd. 39, Nr. Pt 3, Juni 2004 (2004-06), Seiten 339-345, XP009142501, ISSN: 0885-4513
- WANG YUN-QI ET AL: "High-level expression of acidic partner-mediated antimicrobial peptide from tandem genes in Escherichia coli.", APPLIED BIOCHEMISTRY AND BIOTECHNOLOGY 2007 MAY-JUN LNKD- PUBMED:18025552, Bd. 141, Nr. 2-3, Mai 2007 (2007-05), Seiten 203-213, XP009142497, ISSN: 0273-2289
- VASSILEVSKI ALEXANDER A ET AL: "Antimicrobial peptide precursor structures suggest effective production strategies.", RECENT PATENTS ON INFLAMMATION & ALLERGY DRUG DISCOVERY 2008 LNKD- PUBMED:19075992, Bd. 2, Nr. 1, 2008, Seiten 58-63, XP009142549, ISSN: 1872-213X
- LI YIFENG: "Carrier proteins for fusion expression of antimicrobial peptides in Escherichia coli.", BIOTECHNOLOGY AND APPLIED BIOCHEMISTRY SEP 2009 LNKD- PUBMED:19575694, Bd. 54, Nr. 1, 6. Juli 2009 (2009-07-06), Seiten 1-9, XP009142490, ISSN: 1470-8744
- LEE DONG GUN ET AL: "Structure and fungicidal activity of a synthetic antimicrobial peptide, P18, and its truncated peptides", BIOTECHNOLOGY LETTERS, KEW, SURREY, GB, Bd. 26, Nr. 4, 1. Februar 2004 (2004-02-01), Seiten 337-341, XP002602555, ISSN: 0141-5492
- HUEMMERICH D ET AL: "Primary structure elements of spider dragline silks and their contribution to protein solubility", BIOCHEMISTRY, AMERICAN CHEMICAL SOCIETY, US, Bd. 43, Nr. 42, 26. Oktober 2004 (2004-10-26), Seiten 13604-13612, XP002367100, ISSN: 0006-2960, DOI: DOI:10.1021/BI048983Q
- FLORENCE TEULÉ ET AL: "Modifications of spider silk sequences in an attempt to control the mechanical properties of the synthetic fibers", JOURNAL OF MATERIALS SCIENCE, KLUWER ACADEMIC PUBLISHERS, BO, vol. 42, no. 21, 17 July 2007 (2007-07-17) , pages 8974-8985, XP019528790, ISSN: 1573-4803, DOI: 10.1007/S10853-007-1642-6

### SEQUENCE LISTING

<110> BASF SE
<120> Rekombinante Herstellung von Peptiden
<130> M/49358
<160> 77
<170> PatentIn version 3.3
<210> 1
   <211> 14
   <212> PRT
   <213> Artificial
<220>
   <223> Polypeptide
<400> 1
   Gly Ser Ser Ala Ala Ala Ala Ala Ala Ala Ala Ser Gly Pro
   1               5                   10
<210> 2
   <211> 15
   <212> PRT
   <213> Artificial
<220>
   <223> Polypeptide
<400> 2
   Gly Ser Ser Ala Ala Ala Ala Ala Ala Ala Ala Ala Ser Gly Pro
   1               5                   10                  15
<210> 3
   <211> 13
   <212> PRT
   <213> Artificial
<220>
   <223> Polypeptide
<400> 3
   Gly Ser Ala Ala Ala Ala Ala Ala Ala Ala Ser Gly Pro
   1               5                   10
<210> 4
   <211> 13
   <212> PRT
   <213> Artificial
<220>
   <223> Polypeptide
<400> 4
   Gly Ser Val Val Val Val Val Val Val Val Ser Gly Pro
   1 5 10
<210> 5
   <211> 13
   <212> PRT
   <213> Artificial
<220>
   <223> Polypeptide
<400> 5
   Gly Ser Val Val Ala Ala Val Val Ala Ala Ser Gly Pro
   1 5 10
<210> 6
   <211> 18
   <212> PRT
   <213> Artificial
<220>
   <223> P18 Peptide
<400> 6
   Lys Trp Lys Leu Phe Lys Lys Ile Pro Lys Phe Leu His Leu Ala Lys
   1 5 10 15
   Lys Phe
<210> 7
   <211> 19
   <212> PRT
   <213> Artificial
<220>
   <223> Sequence Motif 1
<220>
   <221> MISC_FEATURE
   <222> (1)..(1)
   <223> Xaa = basic amino acid or hydrophobic amino acid different from Pro
<220>
   <221> MISC_FEATURE
   <222> (2)..(2)
   <223> Xaa = basic amino acid or hydrophobic amino acid different from Pro
<220>
   <221> MISC_FEATURE
   <222> (4)..(4)
   <223> Xaa = basic amino acid or hydrophobic amino acid different from Pro
<220>
   <221> MISC_FEATURE
   <222> (5)..(5)
   <223> Xaa = basic amino acid or hydrophobic amino acid different from Pro
<220>
   <221> MISC_FEATURE
   <222> (6)..(6)
   <223> Xaa = basic amino acid or hydrophobic amino acid different from Pro
<220>
   <221> MISC_FEATURE
   <222> (10)..(10)
   <223> Xaa = missing or is Pro or Pro-Pro
<220>
   <221> MISC_FEATURE
   <222> (13)..(13)
   <223> Xaa = basic amino acid or hydrophobic amino acid different from Pro
<220>
   <221> MISC_FEATURE
   <222> (14)..(14)
   <223> Xaa = basic amino acid or hydrophobic amino acid different from Pro
<220>
   <221> MISC_FEATURE
   <222> (15)..(15)
   <223> Xaa = basic amino acid or hydrophobic amino acid different from Pro
<220>
   <221> MISC_FEATURE
   <222> (17)..(17)
   <223> Xaa = basic amino acid or hydrophobic amino acid different from Pro
<400> 7
   Xaa Xaa Lys Xaa Xaa Xaa Lys Ile Pro Xaa Lys Phe Xaa Xaa Xaa Ala
   1               5                   10                  15
   Xaa Lys Phe
<210> 8
   <211> 20
   <212> PRT
   <213> Artificial
<220>
   <223> Sequence Motif 2
<220>
   <221> MISC_FEATURE
   <222> (1)..(1)
   <223> Xaa = Lys, Arg or Phe
<220>
   <221> MISC_FEATURE
   <222> (2)..(2)
   <223> Xaa = Lys or Trp
<220>
   <221> MISC_FEATURE
   <222> (4)..(4)
   <223> Xaa = Leu or Lys
<220>
   <221> MISC_FEATURE
   <222> (5)..(5)
   <223> Xaa = Phe or Leu
<220>
   <221> MISC_FEATURE
   <222> (6)..(6)
   <223> Xaa = Leu or Lys
<220>
   <221> MISC_FEATURE
   <222> (10)..(10)
   <223> Xaa = Pro or missing
<220>
   <221> MISC_FEATURE
   <222> (11)..(11)
   <223> Xaa = Pro or missing
<220>
   <221> MISC_FEATURE
   <222> (14)..(14)
   <223> Xaa = Leu or Lys
<220>
   <221> MISC_FEATURE
   <222> (15)..(15)
   <223> Xaa = His or Lys
<220>
   <221> MISC_FEATURE
   <222> (16)..(16)
   <223> Xaa = Ala, Leu, Val or Ser
<220>
   <221> MISC_FEATURE
   <222> (18)..(18)
   <223> Xaa = Leu or Lys
<400> 8
   Xaa Xaa Lys Xaa Xaa Xaa Lys Ile Pro Xaa Xaa Lys Phe Xaa Xaa Xaa
   1               5                   10                  15
   Ala Xaa Lys Phe
               20
<210> 9
   <211> 19
   <212> PRT
   <213> Artificial
<220>
   <223> Polypeptide
<400> 9
   Lys Trp Lys Leu Phe Lys Lys Ile Pro Pro Lys Phe Leu His Leu Ala
   1               5                   10                  15
   Lys Lys Phe
<210> 10
   <211> 18
   <212> PRT
   <213> Artificial
<220>
   <223> RP18 Peptide
<400> 10
   Arg Trp Lys Leu Phe Lys Lys Ile Pro Lys Phe Leu His Leu Ala Lys
   1               5                   10                  15
   Lys Phe
<210> 11
   <211> 18
   <212> PRT
   <213> Artificial
<220>
   <223> KKFP18 Peptide
<400> 11
   Phe Lys Lys Leu Phe Lys Lys Ile Pro Lys Phe Leu His Ala Ala Lys
   1               5                   10                  15
   Lys Phe
<210> 12
   <211> 18
   <212> PRT
   <213> Artificial
<220>
   <223> KKLP18 Peptide
<400> 12
   Lys Trp Lys Leu Leu Lys Lys Ile Pro Lys Phe Lys Lys Leu Ala Leu
   1 5 10 15
   Lys Phe
<210> 13
   <211> 18
   <212> PRT
   <213> Artificial
<220>
   <223> AP18 Peptide
<400> 13
   Lys Trp Lys Leu Phe Lys Lys Ile Pro Lys Phe Leu His Ala Ala Lys
   1 5 10 15
   Lys Phe
<210> 14
   <211> 18
   <212> PRT
   <213> Artificial
<220>
   <223> KFLP18 Peptide
<400> 14
   Lys Trp Lys Lys Phe Leu Lys Ile Pro Lys Phe Leu His Ala Ala Lys
   1 5 10 15
   Lys Phe
<210> 15
   <211> 18
   <212> PRT
   <213> Artificial
<220>
   <223> KLLP18 Peptide
<400> 15
   Lys Trp Lys Lys Leu Leu Lys Ile Pro Lys Phe Leu His Ala Ala Lys
   1 5 10 15
   Lys Phe
<210> 16
   <211> 14
   <212> PRT
   <213> Artificial
<220>
   <223> Polypeptide
<400> 16
   Phe Glu Glu Ile Ser Glu Phe Leu Gln Ser Leu Glu Glu Phe
   1               5                   10
<210> 17
   <211> 18
   <212> PRT
   <213> Artificial
<220>
   <223> Polypeptide
<400> 17
   Glu Trp Glu Leu Phe Glu Glu Ile Ser Glu Phe Leu Gln Ser Leu Glu
   1               5                   10                  15
   Glu Phe
<210> 18
   <211> 18
   <212> PRT
   <213> Artificial
<220>
   <223> Polypeptide
<400> 18
   Glu Leu Phe Glu Glu Leu Ala Glu Phe Leu Gln Gln Leu Glu Glu Phe
   1               5                   10                  15
   Ile Glu
<210> 19
   <211> 30
   <212> PRT
   <213> Artificial
<220>
   <223> Polypeptide
<400> 19
   Leu Phe Glu Glu Leu Gln Glu Phe Leu Gln Ala Leu Glu Glu Leu Ala
   1               5                   10                  15
   Gln Phe Ala Leu Gln Phe Leu Ala Ala Phe Leu Gln Phe Ser
               20                  25                  30
<210> 20
   <211> 20
   <212> PRT
   <213> Artificial
<220>
   <223> Polypeptide
<400> 20
   Pro Glu Ala His Val Met His Lys Val Ala Pro Arg Pro Gly Gly Gly
   1               5                   10                  15
   Ser Cys Gly Asp
               20
<210> 21
   <211> 456
   <212> DNA
   <213> Artificial
<220>
   <223> Synthetic gene
<400> 21
   ggatccatgg gttccagcgc tgcggcagct gcagcggctg caagtggtcc ggacccggag     60
   gcacacgtta tgcacaaagt agcgccgcgt ccgggtggcg gttcttgtgg tgatccgggt    120
   agctctgcgg ctgcagctgc ggctgcagct tccggtccgg acccggaagc tcacgttatg    180
   cacaaggttg ctccacgccc gggcggtggc agctgcggtg atccaggcag ctctgctgcg    240
   gctgcggcag cggccgcttc tggcccggac ccggaagctc acgttatgca caaagtggct    300
   ccgcgtccgg gtggcggttc ctgcggcgat ccgggttctt ccgctgcagc ggctgcggcc    360
   gcagcgtctg gcccggaccc ggaagcacat gttatgcata aagtagcgcc gcgtccgggc    420
   ggtggctctt gcggtgaccc gggctaatga aagctt                              456
<210> 22
   <211> 304
   <212> PRT
   <213> Artificial
<220>
   <223> Polypeptide
<220>
   <221> MISC_FEATURE
   <222> (1)..(15)
   <223> T7-Tag
<400> 22
   Met Ala Ser Met Thr Gly Gly Gln Gln Met Gly Arg Gly Ser Met Gly
   1               5                   10                  15
   Ser Ser Ala Ala Ala Ala Ala Ala Ala Ala Ser Gly Pro Asp Pro Glu
               20                  25                  30
   Ala His Val Met His Lys Val Ala Pro Arg Pro Gly Gly Gly Ser Cys
           35                  40                  45
   Gly Asp Pro Gly Ser Ser Ala Ala Ala Ala Ala Ala Ala Ala Ser Gly
       50                  55                  60
   Pro Asp Pro Glu Ala His Val Met His Lys Val Ala Pro Arg Pro Gly
   65                  70                  75                  80
   Gly Gly Ser Cys Gly Asp Pro Gly Ser Ser Ala Ala Ala Ala Ala Ala
                   85                  90                  95
   Ala Ala Ser Gly Pro Asp Pro Glu Ala His Val Met His Lys Val Ala
               100                 105                 110
   Pro Arg Pro Gly Gly Gly Ser Cys Gly Asp Pro Gly Ser Ser Ala Ala
           115                 120                 125
   Ala Ala Ala Ala Ala Ala Ser Gly Pro Asp Pro Glu Ala His Val Met
       130                 135                 140
   His Lys Val Ala Pro Arg Pro Gly Gly Gly Ser Cys Gly Asp Pro Gly
   145                 150                 155                 160
   Ser Ser Ala Ala Ala Ala Ala Ala Ala Ala Ser Gly Pro Asp Pro Glu
                   165                 170                 175
   Ala His Val Met His Lys Val Ala Pro Arg Pro Gly Gly Gly Ser Cys
               180                 185                 190
   Gly Asp Pro Gly Ser Ser Ala Ala Ala Ala Ala Ala Ala Ala Ser Gly
           195                 200                 205
   Pro Asp Pro Glu Ala His Val Met His Lys Val Ala Pro Arg Pro Gly
       210                 215                 220
   Gly Gly Ser Cys Gly Asp Pro Gly Ser Ser Ala Ala Ala Ala Ala Ala
   225                 230                 235                 240
   Ala Ala Ser Gly Pro Asp Pro Glu Ala His Val Met His Lys Val Ala
                   245                 250                 255
   Pro Arg Pro Gly Gly Gly Ser Cys Gly Asp Pro Gly Ser Ser Ala Ala
               260                 265                 270
   Ala Ala Ala Ala Ala Ala Ser Gly Pro Asp Pro Glu Ala His Val Met
           275                 280                 285
   His Lys Val Ala Pro Arg Pro Gly Gly Gly Ser Cys Gly Asp Pro Gly
       290                 295                 300
<210> 23
   <211> 21
   <212> PRT
   <213> Artificial
<220>
   <223> P18 Peptide
<400> 23
   Pro Gly Lys Trp Lys Leu Phe Lys Lys Ile Pro Lys Phe Leu His Leu
   1               5                   10                  15
   Ala Lys Lys Phe Gly
               20
<210> 24
   <211> 450
   <212> DNA
   <213> Artificial
<220>
   <223> Synthetic gene
<400> 24
   ggatccatgg gctctagcgc tgcagcggca gctgccgcgg cttctggtcc gggtctgttc     60
   gaagagatct ccgaattcct gcagtctctg gaagagttcg gtggcccggg ttcctctgca    120
   gctgcggctg cagctgcggc aagcggccct gacccaggta aatggaaact gtttaagaaa    180
   attccgaaat tcctgcatct ggctaaaaaa ttcggtgacc cgggttcttc cgctgcggct    240
   gcagctgcag ctgcgtccgg tccgggtctg ttcgaagaaa tctccgaatt cctgcagtct    300
   ctggaagaat tcggcggtcc gggctctagc gctgccgctg cagcggcagc ggcttccggc    360
   ccggacccgg gcaaatggaa actgtttaag aaaatcccga aatttctgca tctggctaaa    420
   aagttcggcg atccgggcta atgaaagctt                                     450
<210> 25
   <211> 300
   <212> PRT
   <213> Artificial
<220>
   <223> Polypeptide
<220>
   <221> MISC_FEATURE
   <222> (1)..(15)
   <223> T7-Tag
<400> 25
   Met Ala Ser Met Thr Gly Gly Gln Gln Met Gly Arg Gly Ser Met Gly
   1               5                   10                  15
   Ser Ser Ala Ala Ala Ala Ala Ala Ala Ala Ser Gly Pro Gly Leu Phe
               20                  25                  30
   Glu Glu Ile Ser Glu Phe Leu Gln Ser Leu Glu Glu Phe Gly Gly Pro
           35                  40                  45
   Gly Ser Ser Ala Ala Ala Ala Ala Ala Ala Ala Ser Gly Pro Asp Pro
       50                  55                  60
   Gly Lys Trp Lys Leu Phe Lys Lys Ile Pro Lys Phe Leu His Leu Ala
   65                  70                  75                  80
   Lys Lys Phe Gly Asp Pro Gly Ser Ser Ala Ala Ala Ala Ala Ala Ala
                   85                  90                  95
   Ala Ser Gly Pro Gly Leu Phe Glu Glu Ile Ser Glu Phe Leu Gln Ser
               100                 105                 110
   Leu Glu Glu Phe Gly Gly Pro Gly Ser Ser Ala Ala Ala Ala Ala Ala
           115                 120                 125
   Ala Ala Ser Gly Pro Asp Pro Gly Lys Trp Lys Leu Phe Lys Lys Ile
       130                 135                 140
   Pro Lys Phe Leu His Leu Ala Lys Lys Phe Gly Asp Pro Gly Ser Ser
   145                 150                 155                 160
   Ala Ala Ala Ala Ala Ala Ala Ala Ser Gly Pro Gly Leu Phe Glu Glu
                   165                 170                 175
   Ile Ser Glu Phe Leu Gln Ser Leu Glu Glu Phe Gly Gly Pro Gly Ser
               180                 185                 190
   Ser Ala Ala Ala Ala Ala Ala Ala Ala Ser Gly Pro Asp Pro Gly Lys
           195                 200                 205
   Trp Lys Leu Phe Lys Lys Ile Pro Lys Phe Leu His Leu Ala Lys Lys
       210                 215                 220
   Phe Gly Asp Pro Gly Ser Ser Ala Ala Ala Ala Ala Ala Ala Ala Ser
   225                 230                 235                 240
   Gly Pro Gly Leu Phe Glu Glu Ile Ser Glu Phe Leu Gln Ser Leu Glu
                   245                 250                 255
   Glu Phe Gly Gly Pro Gly Ser Ser Ala Ala Ala Ala Ala Ala Ala Ala
               260                 265                 270
   Ser Gly Pro Asp Pro Gly Lys Trp Lys Leu Phe Lys Lys Ile Pro Lys
           275                 280                 285
   Phe Leu His Leu Ala Lys Lys Phe Gly Asp Pro Gly
   290 295 300
<210> 26
   <211> 18
   <212> PRT
   <213> Artificial
<220>
   <223> Polypeptide
<400> 26
   Pro Gly Glu Arg Lys Arg Leu Ile Gly Cys Ser Val Met Thr Lys Pro
   1               5                   10                  15
   Ala Gly
<210> 27
   <211> 516
   <212> DNA
   <213> Artificial
<220>
   <223> Synthetic gene
<400> 27
   ggatccatgg gctcttccgc tgcagccgct gcagctgcgg ctgcatccgg tccggaggca     60
   gagccggaag acccgggtga acgtaaacgt ctgatcggtt gttctgtaat gaccaaacct    120
   gctggtgatc cgggctccag cgctgcggct gcggcagctg cagcggcctc tggtccggag    180
   gcggaaccgg aggacccggg tgaacgtaag cgcctgatcg gctgcagcgt gatgaccaaa    240
   ccggctggtg atccgggttc ttccgcggct gcagctgcgg cagctgcagc tagtggtcca    300
   gaagcagaac cagaagaccc gggtgaacgt aaacgtctga ttggttgctc tgttatgact    360
   aaaccggctg gtgacccggg ctcttccgcg gctgccgcgg ctgcggctgc agctagcggc    420
   ccggaagctg aaccggaaga tccgggcgaa cgcaagcgtc tgatcggctg ctccgttatg    480
   actaaaccgg ctggcgaccc gggctaatga aagctt                              516
<210> 28
   <211> 344
   <212> PRT
   <213> Artificial
<220>
   <223> Polypeptide
<220>
   <221> MISC_FEATURE
   <222> (1)..(15)
   <223> T7-Tag
<400> 28
   Met Ala Ser Met Thr Gly Gly Gln Gln Met Gly Arg Gly Ser Met Gly
   1 5 10 15
   Ser Ser Ala Ala Ala Ala Ala Ala Ala Ala Ala Ser Gly Pro Glu Ala
   20 25 30
   Glu Pro Glu Asp Pro Gly Glu Arg Lys Arg Leu Ile Gly Cys Ser Val
   35 40 45
   Met Thr Lys Pro Ala Gly Asp Pro Gly Ser Ser Ala Ala Ala Ala Ala
   50 55 60
   Ala Ala Ala Ala Ser Gly Pro Glu Ala Glu Pro Glu Asp Pro Gly Glu
   65 70 75 80
   Arg Lys Arg Leu Ile Gly Cys Ser Val Met Thr Lys Pro Ala Gly Asp
   85 90 95
   Pro Gly Ser Ser Ala Ala Ala Ala Ala Ala Ala Ala Ala Ser Gly Pro
   100 105 110
   Glu Ala Glu Pro Glu Asp Pro Gly Glu Arg Lys Arg Leu Ile Gly Cys
   115 120 125
   Ser Val Met Thr Lys Pro Ala Gly Asp Pro Gly Ser Ser Ala Ala Ala
   130 135 140
   Ala Ala Ala Ala Ala Ala Ser Gly Pro Glu Ala Glu Pro Glu Asp Pro
   145 150 155 160
   Gly Glu Arg Lys Arg Leu Ile Gly Cys Ser Val Met Thr Lys Pro Ala
   165 170 175
   Gly Asp Pro Gly Ser Ser Ala Ala Ala Ala Ala Ala Ala Ala Ala Ser
   180 185 190
   Gly Pro Glu Ala Glu Pro Glu Asp Pro Gly Glu Arg Lys Arg Leu Ile
   195 200 205
   Gly Cys Ser Val Met Thr Lys Pro Ala Gly Asp Pro Gly Ser Ser Ala
   210 215 220
   Ala Ala Ala Ala Ala Ala Ala Ala Ser Gly Pro Glu Ala Glu Pro Glu
   225 230 235 240
   Asp Pro Gly Glu Arg Lys Arg Leu Ile Gly Cys Ser Val Met Thr Lys
   245 250 255
   Pro Ala Gly Asp Pro Gly Ser Ser Ala Ala Ala Ala Ala Ala Ala Ala
   260 265 270
   Ala Ser Gly Pro Glu Ala Glu Pro Glu Asp Pro Gly Glu Arg Lys Arg
   275 280 285
   Leu Ile Gly Cys Ser Val Met Thr Lys Pro Ala Gly Asp Pro Gly Ser
   290 295 300
   Ser Ala Ala Ala Ala Ala Ala Ala Ala Ala Ser Gly Pro Glu Ala Glu
   305 310 315 320
   Pro Glu Asp Pro Gly Glu Arg Lys Arg Leu Ile Gly Cys Ser Val Met
   325 330 335
   Thr Lys Pro Ala Gly Asp Pro Gly
   340
<210> 29
   <211> 12
   <212> PRT
   <213> Artificial
<220>
   <223> Polypeptide
<400> 29
   Asn Pro Ser Ser Leu Phe Arg Tyr Leu Pro Ser Asp
   1 5 10
<210> 30
   <211> 12
   <212> PRT
   <213> Artificial
<220>
   <223> Polypeptide
<400> 30
   His Gly Gly Gly His Gly His Gly Gly Gly His Gly
   1 5 10
<210> 31
   <211> 12
   <212> PRT
   <213> Artificial
<220>
   <223> Polypeptide
<400> 31
   His Tyr Pro Thr Leu Pro Leu Gly Ser Ser Thr Tyr
   1 5 10
<210> 32
   <211> 12
   <212> PRT
   <213> Artificial
<220>
   <223> Polypeptide
<400> 32
   Ala Leu Ser Pro His Ser Ala Pro Leu Thr Leu Tyr
   1 5 10
<210> 33
   <211> 7
   <212> PRT
   <213> Artificial
<220>
   <223> Polypeptide
<400> 33
   Ser Ala Gly Arg Leu Ser Ala
   1 5
<210> 34
   <211> 7
   <212> PRT
   <213> Artificial
<220>
   <223> Polypeptide
<400> 34
   Thr Leu Pro Asn His Thr Val
   1 5
<210> 35
   <211> 7
   <212> PRT
   <213> Artificial
<220>
   <223> Polypeptide
<400> 35
   His Thr Ser Lys Leu Gly Ile
   1 5
<210> 36
   <211> 16
   <212> PRT
   <213> Artificial
<220>
   <223> Polypeptide
<400> 36
   Met Ser Pro His Pro His Pro Arg His His His Thr Gly Gly Gly Lys
   1 5 10 15
<210> 37
   <211> 17
   <212> PRT
   <213> Artificial
<220>
   <223> Polypeptide
<400> 37
   Glu Ala His Val Met His Lys Val Ala Pro Arg Pro Gly Gly Gly Ser
   1 5 10 15
   Cys
<210> 38
   <211> 18
   <212> PRT
   <213> Artificial
<220>
   <223> Polypeptide
<400> 38
   Ser Ser Lys Lys Ser Gly Ser Tyr Ser Gly Ser Lys Gly Ser Arg Arg
   1 5 10 15
   Ile Leu
<210> 39
   <211> 27
   <212> PRT
   <213> Artificial
<220>
   <223> Polypeptide
<400> 39
   Pro Tyr Ala Tyr Met Lys Ser Arg Asp Ile Glu Ser Ala Gln Ser Asp
   1 5 10 15
   Glu Glu Val Glu Leu Arg Asp Ala Leu Ala Asp
   20 25
<210> 40
   <211> 20
   <212> PRT
   <213> Artificial
<220>
   <223> Polypeptide
<400> 40
   Pro Gly Tyr Gly Tyr Tyr Lys Asn Arg Asn Ala Glu Pro Ala Ala Ala
   1               5                   10                  15
   Glu Ala Val Asp
               20
<210> 41
   <211> 20
   <212> PRT
   <213> Artificial
<220>
   <223> Polypeptide
<400> 41
   Pro Gly Lys Ser Arg Asp Ile Glu Ser Ala Gln Ser Asp Glu Glu Val
   1               5                   10                  15
   Glu Leu Arg Asp
               20
<210> 42
   <211> 17
   <212> PRT
   <213> Artificial
<220>
   <223> Polypeptide
<400> 42
   Pro Gly Lys Ser Arg Asp Ala Glu Pro Ala Ala Ala Gly Glu Glu Val
   1               5                   10                  15
   Asp
<210> 43
   <211> 33
   <212> PRT
   <213> Artificial
<220>
   <223> Polypeptide
<400> 43
   Ser Ser Lys Lys Ser Gly Ser Tyr Ser Gly Ser Lys Gly Ser Arg Arg
   1               5                   10                  15
   Ile Leu Gly Gly Gly Asn Pro Ser Ser Leu Phe Arg Tyr Leu Pro Ser
               20                  25                  30
   Asp
<210> 44
   <211> 27
   <212> PRT
   <213> Artificial
<220>
   <223> Polypeptide
<400> 44
   Met Ser Pro His Pro His Pro Arg His His His Thr Gly Gly Gly Asn
   1               5                   10                  15
   Pro Ser Ser Leu Phe Arg Tyr Leu Pro Ser Asp
               20                  25
<210> 45
   <211> 31
   <212> PRT
   <213> Artificial
<220>
   <223> Polypeptide
<400> 45
   Asn Pro Ser Ser Leu Phe Arg Tyr Leu Pro Ser Asp Gly Gly Gly Arg
   1               5                   10                  15
   Arg Glu Glu Trp Trp Asp Asp Arg Arg Glu Glu Trp Trp Asp Asp
               20                  25                  30
<210> 46
   <211> 27
   <212> PRT
   <213> Artificial
<220>
   <223> Polypeptide
<400> 46
   Met Ser Pro His Pro His Pro Arg His His His Thr Gly Gly Gly His
   1 5 10 15
   Gly Gly Gly His Gly His Gly Gly Gly His Gly
   20 25
<210> 47
   <211> 33
   <212> PRT
   <213> Artificial
<220>
   <223> Polypeptide
<400> 47
   Ser Ser Lys Lys Ser Gly Ser Tyr Ser Gly Ser Lys Gly Ser Arg Arg
   1 5 10 15
   Ile Leu Gly Gly Gly His Gly Gly Gly His Gly His Gly Gly Gly His
   20 25 30
   Gly
<210> 48
   <211> 33
   <212> PRT
   <213> Artificial
<220>
   <223> Polypeptide
<400> 48
   Ser Ser Lys Lys Ser Gly Ser Tyr Ser Gly Ser Lys Gly Ser Arg Arg
   1 5 10 15
   Ile Leu Gly Gly Gly His Tyr Pro Thr Leu Pro Leu Gly Ser Ser Thr
   20 25 30
   Tyr
<210> 49
   <211> 28
   <212> PRT
   <213> Artificial
<220>
   <223> Polypeptide
<400> 49
   Ser Ser Lys Lys Ser Gly Ser Tyr Ser Gly Ser Lys Gly Ser Arg Arg
   1               5                   10                  15
   Ile Leu Gly Gly Gly Ser Ala Gly Arg Leu Ser Ala
               20                  25
<210> 50
   <211> 16
   <212> PRT
   <213> Artificial
<220>
   <223> Polypeptide
<400> 50
   Arg Arg Glu Glu Trp Trp Asp Asp Arg Arg Glu Glu Trp Trp Asp Asp
   1               5                   10                  15
<210> 51
   <211> 21
   <212> PRT
   <213> Artificial
<220>
   <223> Polypeptide
<400> 51
   Met Lys Gln Leu Ala Asp Ser Leu Met Gln Leu Ala Arg Gln Val Ser
   1               5                   10                  15
   Arg Leu Glu Ser Ala
               20
<210> 52
   <211> 21
   <212> PRT
   <213> Artificial
<220>
   <223> Polypeptide
<400> 52
   Met Lys Gln Leu Ala Asp Ser Leu His Gln Leu Ala Arg Gln Val Ser
   1               5                   10                  15
   Arg Leu Glu His Ala
               20
<210> 53
   <211> 28
   <212> PRT
   <213> Artificial
<220>
   <223> Polypeptide
<400> 53
   Leu Met Gln Leu Ala Arg Gln Met Lys Gln Leu Ala Asp Ser Leu Met
   1               5                   10                  15
   Gln Leu Ala Arg Gln Val Ser Arg Leu Glu Ser Ala
               20                  25
<210> 54
   <211> 21
   <212> PRT
   <213> Artificial
<220>
   <223> Polypeptide
<400> 54
   Met Lys Glu Leu Ala Asp Ser Leu Met Gln Leu Ala Arg Gln Val Asp
   1               5                   10                  15
   Arg Leu Glu Ser Ala
               20
<210> 55
   <211> 21
   <212> PRT
   <213> Artificial
<220>
   <223> Polypeptide
<400> 55
   Met Lys Gln Leu Ala Asp Ser Leu His Gln Leu Ala His Gln Val Ser
   1               5                   10                  15
   His Leu Glu His Ala
               20
<210> 56
   <211> 9
   <212> PRT
   <213> Artificial
<220>
   <223> Polypeptide
<400> 56
   Pro His Phe Arg Phe Ser Phe Ser Pro
   1               5
<210> 57
   <211> 9
   <212> PRT
   <213> Artificial
<220>
   <223> Polypeptide
<400> 57
   Pro His Phe Ser Phe Ser Phe Ser Pro
   1               5
<210> 58
   <211> 9
   <212> PRT
   <213> Artificial
<220>
   <223> Polypeptide
<400> 58
   Pro Ser Phe Arg Phe Ser Phe Ser Pro
   1               5
<210> 59
   <211> 21
   <212> PRT
   <213> Artificial
<220>
   <223> Polypeptide
<400> 59
   Met Glu Glu Leu Ala Asp Ser Leu Glu Glu Leu Ala Arg Gln Val Glu
   1               5                   10                  15
   Glu Leu Glu Ser Ala
               20
<210> 60
   <211> 21
   <212> PRT
   <213> Artificial
<220>
   <223> Polypeptide
<400> 60
   Met Lys Lys Leu Ala Asp Ser Leu Lys Lys Leu Ala Arg Gln Val Lys
   1 5 10 15
   Lys Leu Glu Ser Ala
   20
<210> 61
   <211> 9
   <212> PRT
   <213> Artificial
<220>
   <223> Polypeptide
<400> 61
   Pro His Phe His Phe Ser Phe Ser Pro
   1 5
<210> 62
   <211> 9
   <212> PRT
   <213> Artificial
<220>
   <223> Polypeptide
<400> 62
   Pro His Phe Ser Phe His Phe Ser Pro
   1 5
<210> 63
   <211> 21
   <212> PRT
   <213> Artificial
<220>
   <223> Polypeptide
<400> 63
   Met Lys Gln Leu Ala Asp Ser Leu His Gln Leu Ala His Lys Val Ser
   1 5 10 15
   His Leu Glu His Ala
   20
<210> 64
   <211> 21
   <212> PRT
   <213> Artificial
<220>
   <223> Polypeptide
<400> 64
   Glu Ile Ser Ala Leu Glu Lys Glu Ile Ser Ala Leu Glu Lys Glu Ile
   1 5 10 15
   Ser Ala Leu Glu Lys
   20
<210> 65
   <211> 21
   <212> PRT
   <213> Artificial
<220>
   <223> Polypeptide
<400> 65
   Lys Ile Ser Ala Leu Lys Glu Lys Ile Ser Ala Leu Lys Glu Lys Ile
   1 5 10 15
   Ser Ala Leu Lys Glu
   20
<210> 66
   <211> 16
   <212> PRT
   <213> Artificial
<220>
   <223> Polypeptide
<400> 66
   Arg Ala Asp Ala Arg Ala Asp Ala Arg Ala Asp Ala Arg Ala Asp Ala
   1 5 10 15
<210> 67
   <211> 21
   <212> PRT
   <213> Artificial
<220>
   <223> Polypeptide
<400> 67
   Val Lys Val Lys Val Lys Val Lys Val Gly Pro Pro Thr Lys Val Lys
   1 5 10 15
   Val Lys Val Lys Val
   20
<210> 68
   <211> 6
   <212> PRT
   <213> Artificial
<220>
   <223> Peptide
<400> 68
   Glu Ala Glu Pro Glu Asp
   1 5
<210> 69
   <211> 22
   <212> PRT
   <213> Artificial
<220>
   <223> Peptide
<400> 69
   Pro Gly Lys Trp Lys Leu Phe Lys Lys Ile Pro Lys Phe Leu His Leu
   1 5 10 15
   Ala Lys Lys Phe Gly Asp
   20
<210> 70
   <211> 19
   <212> PRT
   <213> Artificial
<220>
   <223> Peptide
<400> 70
   Pro Gly Glu Arg Lys Arg Leu Ile Gly Cys Ser Val Met Thr Lys Pro
   1 5 10 15
   Ala Gly Asp
<210> 71
   <211> 22
   <212> PRT
   <213> Artificial
<220>
   <223> Peptide
<400> 71
   Pro Gly Lys Trp Lys Leu Phe Lys Lys Ile Pro Lys Phe Leu His Leu
   1               5                   10                  15
   Ala Lys Lys Phe Gly Asn
               20
<210> 72
   <211> 15
   <212> PRT
   <213> Artificial
<220>
   <223> Peptide
<400> 72
   Glu Arg Lys Arg Leu Ile Gly Cys Ser Val Met Thr Lys Pro Ala
   1               5                   10                  15
<210> 73
   <211> 12
   <212> PRT
   <213> Artificial
<220>
   <223> Polypeptide
<400> 73
   Gly Ala Ala Ala Ala Ala Ala Ala Ala Ser Gly Pro
   1               5                   10
<210> 74
   <211> 456
   <212> DNA
   <213> Artificial
<220>
   <223> Synthetic gene
<400> 74
   ggatccatgg gcgctgcagc ggcagctgcc gcggcttctg gtccgggtga gtgggagctg     60
   ttcgaagaga tcagcgaatt cctgcagtct ctggaagagt tcggtggccc gggttcctct    120
   gctgctgcgg ctgcagctgc ggcaggcccg ggcgacccag gtaaatggaa actgtttaag    180
   aaaattccga aattcctgca tctggctaaa aaattcggtg acccgggttc ctctgctgcg    240
   gctgcagctg cagctgcgtc cggtccgggt gaatgggaac tgttcgaaga aatctccgaa    300
   ttcctgcagt ctctggaaga attcggcggt ccgggcgctg ccgctgcagc ggcagcggct    360
   ggtcctggcg acccgggcaa atggaaactg tttaagaaaa tcccgaaatt tctgcatctg    420
   gctaaaaagt tcggcgatcc gggctaatga aagctt                              456
<210> 75
   <211> 304
   <212> PRT
   <213> Artificial
<220>
   <223> Polypeptide
<400> 75
   Met Ala Ser Met Thr Gly Gly Gln Gln Met Gly Arg Gly Ser Met Gly
   1               5                   10                  15
   Ala Ala Ala Ala Ala Ala Ala Ala Ser Gly Pro Gly Glu Trp Glu Leu
               20                  25                  30
   Phe Glu Glu Ile Ser Glu Phe Leu Gln Ser Leu Glu Glu Phe Gly Gly
           35                  40                  45
   Pro Gly Ser Ser Ala Ala Ala Ala Ala Ala Ala Ala Gly Pro Gly Asp
       50                  55                  60
   Pro Gly Lys Trp Lys Leu Phe Lys Lys Ile Pro Lys Phe Leu His Leu
   65                  70                  75                  80
   Ala Lys Lys Phe Gly Asp Pro Gly Ser Ser Ala Ala Ala Ala Ala Ala
                   85                  90                  95
   Ala Ala Ser Gly Pro Gly Glu Trp Glu Leu Phe Glu Glu Ile Ser Glu
               100                 105                 110
   Phe Leu Gln Ser Leu Glu Glu Phe Gly Gly Pro Gly Ala Ala Ala Ala
           115                 120                 125
   Ala Ala Ala Ala Gly Pro Gly Asp Pro Gly Lys Trp Lys Leu Phe Lys
       130                 135                 140
   Lys Ile Pro Lys Phe Leu His Leu Ala Lys Lys Phe Gly Asp Pro Gly
   145                 150                 155                 160
   Ala Ala Ala Ala Ala Ala Ala Ala Ser Gly Pro Gly Glu Trp Glu Leu
                   165                 170                 175
   Phe Glu Glu Ile Ser Glu Phe Leu Gln Ser Leu Glu Glu Phe Gly Gly
               180                 185                 190
   Pro Gly Ser Ser Ala Ala Ala Ala Ala Ala Ala Ala Gly Pro Gly Asp
           195                 200                 205
   Pro Gly Lys Trp Lys Leu Phe Lys Lys Ile Pro Lys Phe Leu His Leu
       210                 215                 220
   Ala Lys Lys Phe Gly Asp Pro Gly Ser Ser Ala Ala Ala Ala Ala Ala
   225                 230                 235                 240
   Ala Ala Ser Gly Pro Gly Glu Trp Glu Leu Phe Glu Glu Ile Ser Glu
                   245                 250                 255
   Phe Leu Gln Ser Leu Glu Glu Phe Gly Gly Pro Gly Ala Ala Ala Ala
               260                 265                 270
   Ala Ala Ala Ala Gly Pro Gly Asp Pro Gly Lys Trp Lys Leu Phe Lys
           275                 280                 285
   Lys Ile Pro Lys Phe Leu His Leu Ala Lys Lys Phe Gly Asp Pro Gly
       290                 295                 300
<210> 76
   <211> 438
   <212> DNA
   <213> Artificial
<220>
   <223> Synthetic gene
<400> 76
   ggatccatgg gcgctgcagc ggcagctgcc gcggcttctg gtccgggtga gtgggagctg      60
   ttcgaagaga tcagcgaatt cctgcagtct ctggaagagt tcggtggccc gggttcctct     120
   gctgctgcgg ctgcagctgc ggcaggccca ggcgacaaat ggaaactgtt taagaaaatt     180
   ccgaaattcc tgcatctggc taaaaaattc gacccgggtt cctctgctgc ggctgcagct     240
   gcagctgcgt ccggtccggg tgaatgggaa ctgttcgaag aaatctccga attcctgcag     300
   tctctggaag aattcggcgg tccgggcgct gccgctgcag cggcagcggc tggtcctggc     360
   gacaaatgga aactgtttaa gaaaatcccg aaatttctgc atctggctaa aaagttcgat     420
   ccgggctaat gaaagctt                                                   438
<210> 77
   <211> 278
   <212> PRT
   <213> Artificial
<220>
   <223> Polypeptide
<400> 77
   Met Gly Ala Ala Ala Ala Ala Ala Ala Ala Ser Gly Pro Gly Glu Trp
   1               5                   10                  15
   Glu Leu Phe Glu Glu Ile Ser Glu Phe Leu Gln Ser Leu Glu Glu Phe
               20                  25                  30
   Gly Gly Pro Gly Ser Ser Ala Ala Ala Ala Ala Ala Ala Ala Gly Pro
           35                  40                  45
   Gly Asp Lys Trp Lys Leu Phe Lys Lys Ile Pro Lys Phe Leu His Leu
       50                  55                  60
   Ala Lys Lys Phe Asp Pro Gly Ser Ser Ala Ala Ala Ala Ala Ala Ala
   65                  70                  75                  80
   Ala Ser Gly Pro Gly Glu Trp Glu Leu Phe Glu Glu Ile Ser Glu Phe
                   85                  90                  95
   Leu Gln Ser Leu Glu Glu Phe Gly Gly Pro Gly Ala Ala Ala Ala Ala
               100                 105                 110
   Ala Ala Ala Gly Pro Gly Asp Lys Trp Lys Leu Phe Lys Lys Ile Pro
           115                 120                 125
   Lys Phe Leu His Leu Ala Lys Lys Phe Asp Pro Gly Ala Ala Ala Ala
       130                 135                 140
   Ala Ala Ala Ala Ser Gly Pro Gly Glu Trp Glu Leu Phe Glu Glu Ile
   145                 150                 155                 160
   Ser Glu Phe Leu Gln Ser Leu Glu Glu Phe Gly Gly Pro Gly Ser Ser
                   165                 170                 175
   Ala Ala Ala Ala Ala Ala Ala Ala Gly Pro Gly Asp Lys Trp Lys Leu
               180                 185                 190
   Phe Lys Lys Ile Pro Lys Phe Leu His Leu Ala Lys Lys Phe Asp Pro
           195                 200                 205
   Gly Ser Ser Ala Ala Ala Ala Ala Ala Ala Ala Ser Gly Pro Gly Glu
       210                 215                 220
   Trp Glu Leu Phe Glu Glu Ile Ser Glu Phe Leu Gln Ser Leu Glu Glu
   225                 230                 235                 240
   Phe Gly Gly Pro Gly Ala Ala Ala Ala Ala Ala Ala Ala Gly Pro Gly
                   245                 250                 255
   Asp Lys Trp Lys Leu Phe Lys Lys Ile Pro Lys Phe Leu His Leu Ala
               260                 265                 270
   Lys Lys Phe Asp Pro Gly
           275

## Beschreibung

Die vorliegende Erfindung betrifft repetitive selbstassemblierende Vorläuferproteine, dafür kodierende Nukleinsäuresequenzen und Expressionskonstrukte, sowie Verfahren zur rekombinanten Herstellung von Peptiden unter Verwendung von solchen Vorläuferproteinen.

### Hintergrund der Erfindung

Synthetische repetitive Proteine sind bekannt, z.B. aus der WO 2008/155304 A1. WO 2006/008163 A3, WO 2007/ 025719 A1, Hümmerich et al. (Biochem. 43; 13604-13612 (2004)) und Teule et al. (J. Mat. Sci. 42; 8974-8985 (2007)) beschreiben Spinnseidenproteine, welche aus repetitiven Untereinheiten bestehen. WO 2007/082936 A1 beschreibt amphiphile selbstassemblierende Proteine zur Formulierung von schwer wasserlöslichen Effektstoffen, wie z.B. synthetische Proteine, welche auf Wiederholungseinheiten von natürlichen Seidenproteinen basieren.

Es sind unterschiedliche Methoden zur biotechnologischen Herstellung von Peptiden bekannt. Da die Stabilität von kurzen Polypeptidketten in mikrobiellen Wirtszellen in der Regel gering ist und die freien Peptide unter Umständen toxisch auf den Wirtsorganismus wirken können (z.B. antimikrobielle Peptide), werden bei den meisten Methoden größere Vorläuferproteine gebildet, aus denen das Peptid nach der Aufreinigung des Vorläuferproteins herausgeschnitten wird.

Eine Möglichkeit, ein stabiles Vorläuferprotein zu erhalten, besteht darin, ein Peptid zusammen mit einem stabilen Protein als Fusionsprotein zu exprimieren. Die Eigenschaften des Fusionsproteins, die einen großen Einfluss auf nachfolgende Aufarbeitungsschritte haben, werden weitgehend unabhängig von der Peptidsequenz von dem Fusionspartner bestimmt und sind damit gut kontrollierbar und für die Herstellung von Peptiden mit unterschiedlichen Sequenzen geeignet.

Die WO 2008/085543 beschreibt ein spezielles Verfahren zur Herstellung von Proteinen und Peptiden mit Hilfe eines Fusionsproteins. Dieses Fusionsprotein enthält neben der gewünschten Peptidsequenz einen Fusionspartner, der dafür sorgt, dass das Fusionsprotein ein inverses Phasenübergangsverhalten zeigt. Dieses Verhalten erlaubt einerseits eine einfache und kostengünstige Aufreinigung des Fusionsproteins aus dem zellulären Kontext. Andererseits kann die Abtrennung des Fusionspartners nach der proteolytischen Abspaltung des Peptids ebenfalls einfach und kostengünstig durchgeführt werden. Während ein Fusionsprotein oft mit guten Ausbeuten erhalten werden kann, ist der Anteil des Peptids am Vorläuferprotein in der Regel gering und somit die Effizienz des Prozesses nicht optimal.

In einem anderen Ansatz werden repetitive Vorläuferproteine, die mehrere Kopien des gewünschten Peptids enthalten, rekombinant hergestellt. Die WO 03/089455 beschreibt die Herstellung multimerer Vorläuferproteine, aus denen die gewünschten Peptidsequenzen, die antimikrobielle Eigenschaften aufweisen, durch Säurespaltung herausgeschnitten werden.

Es gibt eine Reihe weiterer publizierter Ansätze (Beispiele: Metlitskaya et al. Biotechnol Appl. Biochem 39; 339-345 (2004); Wang & Cai Appl. Biochem and Biotechnol. 141; 203-213 (2007)), mit denen gezeigt wurde, dass Peptidsequenzen oder Familien von Peptidsequenzen nach einem bestimmten Verfahren mit Hilfe von repetitiven Vorläuferproteinen hergestellt werden können. Teilweise wurde die Verwendung von speziellen Hilfssequenzen beschrieben, die zwischen den Wiederholungen der gewünschten Peptidsequenzen angeordnet sind. Insbesondere wurden anionische Hilfssequenzen vorgeschlagen, die die schädigende Wirkung von kationischen antimikrobiellen Peptidsequenzen innerhalb eines repetitiven Vorläuferproteins auf die Wirtszelle verringern sollen (vgl. z.B. WO 00/31279, Lee et al. Prot. Expr. Purificat. 12; 53-60 (1998); WO 98/54336 A1; Kim et al. Appl. Microbiol. Biotechnol. 72; 330-338 (2006) und US 2003/0219854). Während bei diesem repetitiven Ansatz der Anteil der gewünschten Peptidsequenz am Vorläuferprotein höher ist, als bei Fusionsproteinen, werden die Eigenschaften des repetitiven Vorläuferproteins stark von der Sequenz des gewünschten kationischen Peptids beeinflusst.

Den Erfindern ist bisher kein Verfahren bekannt, bei dem mit Hilfe von repetitiven Vorläuferproteinen beliebige Peptidsequenzen nach einem einfachen, günstigen und effizient durchführbaren Protokoll hergestellt werden können.

Diverse antimikrobielle Peptide sind in der Literatur bereits beschrieben und in Reviews zusammengefasst (Hancock, R.E.W. und Lehrer, R. 1998 in Trends in Biotechnology, 16: 82-88; Hancock, R.E.W. und Sahl, H.G. 2006 in Nature Biotechnology, 24: 1551-1557; Lee et al. 2004 in Biotechnology Letters 26: 337-341); auch deren rekombinante Herstellung (Vassilevski et al. 2008 in Recent Patents on Inflammation & Allergy Drug Discovery 2: 58-63).

Fusionspeptide, die zwei wirksame Peptide in sich vereinen, sind in der Literatur ebenfalls beschrieben. Wade *et al.* berichten über die antibakterielle Wirkung diverser Fusionen aus Cecropin A aus *Hyalophora cecropia* und dem Bienengift Melittin (Wade, D. et al., 1992, International Journal of Peptide and Protein Research, 40: 429-436). Shin *et al.* beschreiben die antibakterielle Wirkung eines Fusionspeptids aus Cecropin A aus *Hyalophora cecropia* und Magainin 2 aus *Xenopus laevis,* bestehend aus 20 Aminosäuren. Cecropin A besteht aus 37 Aminosäuren und zeigt Aktivität gegen gramnegative Bakterien, aber geringere Aktivität gegen grampositive Bakterien. Magainin 2 besteht aus 23 Aminosäuren und ist aktiv gegen Bakterien aber auch Tumorzelllinien. Im Vergleich zur Fusion aus Cecropin A und Melittin zeigt diese Fusion eine deutlich geringere hämolytische Aktivität bei vergleichbarer antibakterieller Wirkung (Shin, S.Y. Kang, J.H., Lee, M.K., Kim, S.Y., Kim, Y., Hahm, K.S., 1998, Biochemistry and Molecular Biology International, 44: 1119-1126). US 2003/0096745 A1 und US 6,800,727 B2 beanspruchen diese Fusionspeptide, bestehend aus 20 Aminosäuren und Varianten dieser Fusion, die durch den Austausch von Aminosäuren, insbesondere von positiv geladenen Aminosäuren und hydrophoben Aminosäuren, stärker positiv geladen und hydrophober sind.

Weitere Entwicklungen dieses Cecropin-A-Magainin-2-Fusionspeptids beschreiben Shin *et al.* 1999. Hierbei zeigte sich, dass das Peptid mit der SEQ ID NO:6 eine geringere hämolytische Aktivität im Vergleich zur Ausgangsfusion aufwies, die antibakterielle Aktivität gegenüber *Escherichia coli* und *Bacillus subtilis* jedoch nicht beeinträchtigt wurde (Shin et al. 1999 Journal of Peptide Research, 53: 82-90).

### Zusammenfassung der Erfindung

Aufgabe der vorliegenden Erfindung war es daher, ein vielseitig anwendbares Verfahren zur Herstellung von Peptiden mit Hilfe repetitiver Vorläuferproteine bereitzustellen.

Gelöst wurde diese Aufgabe durch einen neuartigen Ansatz zur biotechnologischen Herstellung von Peptiden, bei denen repetitive Vorläuferproteine hergestellt werden, die einen hohen Anteil der gewünschten Peptidsequenz enthalten und die Hilfssequenzen enthalten, welche die Eigenschaften des Vorläuferproteins in vorhersagbarer Weise dominieren. Dieses Verfahren kann für die Herstellung unterschiedlicher Peptidsequenzen verwendet werden, ohne die Expressionsbedingungen des Vorläufermoleküls oder die anschließende Aufarbeitung jeweils für unterschiedliche Peptidsequenzen grundlegend neu etablieren zu müssen. Außerdem können Peptide hergestellt werden, bei denen bisher verwendete Methoden nicht effizient sind.

### Figurenbeschreibung

In den beiliegenden Figuren zeigt
Figur 1 eine Helical-Wheel-Darstellung von Aminosäuresequenzen, als Projektion einer alpha-Helixstruktur. Die im repetitiven Vorläuferprotein enthaltene Aminosäuresequenz A1 - A7 (A) wird auf einem Kreis dargestellt (B). Aus dieser Anordnung wird die Lage der Aminosäuren in einer alpha-Helix sichtbar;
Figur 2 das Reversed Phase Chromatogramm des Peptids "ZnO" nach Säurespaltung;
Figur 3 das Massenspektrum des Peptids "ZnO" nach Säurespaltung und Reversed Phase HPLC; die gezeigten Zahlen geben den m/z-Wert des jeweiligen monoisotopischen Peaks an;
Figur 4 das Reversed Phase Chromatogramm des Peptids "P18" nach Säurespaltung und Kationenaustauschchromatographie;
Figur 5 das Massenspektrum des Peptids "P18" nach Säurespaltung, Kationenaustauschchromatographie und Reversed Phase HPLC; die gezeigten Zahlen geben den m/z-Wert des jeweiligen monoisotopischen Peaks an;
Figur 6 das Reversed Phase Chromatogramm des Peptids "Min" nach Säurespaltung;
Figur 7 das Massenspektrum des Peptids "Min" nach Säurespaltung und Reversed Phase HPLC; die gezeigten Zahlen geben den m/z-Wert des jeweiligen monoisotopischen Peaks an.
Figur 8 das Reversed Phase Chromatogramm des Peptids SEQ ID NO:6 nach Säurespaltung und Kationenaustauschchromatographie;
Figur 9 das Massenspektrum des Peptids SEQ ID NO:6 nach Säurespaltung, Kationenaustauschchromatographie und Reversed Phase HPLC; die gezeigten Zahlen geben den m/z-Wert des jeweiligen monoisotopischen Peaks an;
Figur 10 das HPLC-Kationenaustauschchromatogramm des Peptids "P18" vor und nach Amidierung entsprechend Beispiel 6; zum Vergleich ist das Chromatogramm eins chemisch synthetisierten und amidierten Referenz-Peptids mit der Sequenz des Peptids "P18" dargestellt;
Figur 11 das HPLC-Kationenaustauschchromatogramm des Peptids "P18" vor und nach Amidierung entsprechend Beispiel 7; zum Vergleich ist das Chromatogramm eins chemisch synthetisierten und amidierten Referenz-Peptids mit der Sequenz des Peptids "P18" dargestellt;

### Bevorzugte Ausführungsformen

Die Erfindung betrifft insbesondere folgende Ausführungsformen:
1. Vorläuferprotein, insbesondere ein synthetisches, insbesondere rekombinant hergestelltes Vorläuferprotein, umfassend eine spaltbare repetitive Abfolge von Wiederholungseinheiten aus gewünschten Peptid (Pep)-Elementen und Hilfspeptid (Aux)-Elementen, der allgemeinen Formel
   (Pep-Aux)ₓ oder
   (Aux-Pep)ₓ
   worin x >1 ist, wobei
   die Aux-Elemente gleich oder verschieden sind und Aminosäuresequenzelemente umfassen, die dem Vorläuferprotein selbstassemblierende Eigenschaften verleihen, wobei das Aux-Element ein selbstassemblierendes Peptid ((SA)-Element) umfasst
   wobei das SA-Element wenigstens eines der folgenden Sequenzmotive enthält:
   Aₙ (Motiv 1)
   (GA)ₘ (Motiv 2)
   Vₙ (Motiv 3)
   (VA)ₘ (Motiv 4)
   (WAA)ₒ (Motiv 5)
   worin A für Alanin, G für Glycin, V für Valin, n für einen ganzzahligen Wert von 8 bis 12, m für einen ganzzahligen Wert von 4 bis 10, und o für einen ganzzahligen Wert von 2 bis 6 steht; und
   die Pep-Elemente gleich oder verschieden sind und die Aminosäuresequenz gleicher oder verschiedener Peptidmoleküle mit einer Sequenzlänge von 5-70 Aminosäureresten umfassen, und die Pep-Elemente von Spaltsequenzen flankiert sind, die eine spezifische Herausspaltung der Pep-Elemente aus dem Vorläuferprotein ermöglichen, wobei
   die Elemente Pep und Aux miteinander peptidisch verknüpft sind und die peptidische Verknüpfung chemisch spezifisch spaltbar ist, wobei die Spaltsequenzen ein Sequenzmotiv, ausgewählt unter NG und DP, umfassen.
2. Vorläuferprotein, umfassend eine spaltbare repetitive Abfolge von gewünschten Peptid (Pep)-Elementen und Hilfspeptid (Aux)-Elementen, der allgemeinen Formel
   (Pep-Aux)ₓ oder
   (Aux-Pep)ₓ
   worin x >1 ist, wobei
   die Aux-Elemente gleich oder verschieden sind und Aminosäuresequenzelemente umfassen, die dem Vorläuferprotein selbstassemblierende Eigenschaften verleihen, wobei das Aux-Element ein selbstassemblierendes Peptid ((SA)-Element) umfasst
   wobei das SA-Element wenigstens eines der folgenden Sequenzmotive enthält:
   Aₙ (Motiv 1)
   (GA)ₘ (Motiv 2)
   Vₙ (Motiv 3)
   (VA)ₘ (Motiv 4)
   (WAA)ₒ (Motiv 5)
   worin A für Alanin, G für Glycin, V für Valin, n für einen ganzzahligen Wert von 8 bis 12, m für einen ganzzahligen Wert von 4 bis 10, und o für einen ganzzahligen Wert von 2 bis 6 steht; und
   die Pep-Elemente gleich oder verschieden sind und die Aminosäuresequenz gleicher oder verschiedener Peptidmoleküle mit einer Sequenzlänge von 5-70 Aminosäureresten umfassen, und die Pep-Elemente von Spaltsequenzen flankiert sind, die eine spezifische Herausspaltung der Pep-Elemente aus dem Vorläuferprotein ermöglichen, wobei
   die Elemente Pep und Aux miteinander peptidisch verknüpft sind und die peptidische Verknüpfung chemisch oder enzymatisch spezifisch spaltbar ist, wobei
   a) das Pep-Element eine Aminosäuresequenz, ausgewählt unter den kationischen Aminosäuresequenzen SEQ ID NO: 6 bis SEQ ID NO:15, SEQ ID NO: 23, SEQ ID NO: 26 und SEQ ID NO: 69 bis SEQ ID NO: 72 umfasst, oder
   b) das Pep-Element eine Aminosäuresequenz, ausgewählt unter den Aminosäuresequenzen SEQ ID NO: 20 oder SEQ ID NO: 29 bis 67 umfasst.
3. Ein Vorläuferprotein nach einer der vorhergehenden Ausführungsformen, worin die Elemente Pep und Aux miteinander peptidisch direkt oder über eine spaltbare Peptidsequenz verknüpft sind und die peptidische Verknüpfung chemisch spezifisch, d.h. ausschließlich oder im Wesentlichen an einer definierten Aminosäure oder Abfolge von Aminosäuren einer Sequenz spaltbar ist.
4. Ein Vorläuferprotein nach einer der vorhergehenden Ausführungsformen, welches selbstassemblierende Eigenschaften besitzt, so dass es spontan, d.h. von selbst, oder induzierbar, stabile, nicht-kovalente Assoziate bildet, die unter Standardbedingungen, wie insbesondere durch 0,2 M NaOH innerhalb einer Stunde oder von 2 M Harnstoff oder 1 M Guanidiniumhydrochlorid jeweils innerhalb von 10 min bei Raumtemperatur nicht auflösbar sind. Ist wenigstens eines dieser drei genannten Kriterien erfüllt, so liegt ein erfindungsgemäßes stabiles Assoziat vor.
5. Ein Vorläuferprotein nach einer der vorhergehenden Ausführungsformen, wobei wenigstens ein Aux-Element ein selbstassemblierendes Peptid (SA)-Element umfasst, wobei das SA-Element wenigstens ein Sequenzmotiv von mindestens 8, wie z.B. 8-10, 8-12, 8-14, 8-16. 8-18 oder 8-20, zusammenhängenden Aminosäuren enthält, das mindestens 50%, wie z.B. 50-100%, 60-90% oder 70-80%, Alanin-Reste, mindestens 50%, wie z.B. 50-100%, 60-90% oder 70-80%, Valin-Reste oder mindestens 50%, wie z.B. 50-100%, 60-90% oder 70-80%, GlutaminReste enthält, oder zu mindestens 80% aus wenigstens einem dieser Reste besteht; Das SA-Element kann z.B. insbesondere wenigstens eines der folgenden Sequenzmotive enthalten:
   Aₙ (Motiv 1)
   (GA)ₘ (Motiv 2)
   Vₙ (Motiv 3)
   (VA)ₘ (Motiv 4)
   (WAA)ₒ (Motiv 5),
   wobei insbesondere n = 8 - 10, m = 4 - 8, und o=2-4 ist, wie z.B. n = 8 - 9, m = 6 - 7 und o= 2-3 ist.
   Obige SA-Sequenzen können C- und oder N-terminal um jeweils 1 bis 3 weitere beliebige Aminosäurereste elongiert sein. Beispiele für geeignete N-terminale Verlängerungen sind die Sequenzmotive "G-", "GS-", "GAG-", "GPG-", "GPS-", "GAS-", "GQQ-" und "GSS-"; Beispiele für geeignete C-terminale Verlängerungen umfassen das Sequenzmotiv "-SGP", "-GGA", "-GPG", "-SGA", "-GGQ" , "-GGY" und "-GGL".
6. Ein Vorläuferprotein nach Ausführungsform 5, worin das SA-Element eine Aminosäuresequenz, ausgewählt unter den Aminosäuresequenzen SEQ ID NO: 1 bis SEQ ID NO:5, oder SEQ ID NO:73 umfasst.
7. Ein Vorläuferprotein nach einer der vorhergehenden Ausführungsformen, wobei wenigstens ein Aux-Peptid außerdem ein Schutzpeptid (SU)-Element umfasst.
8. Ein Vorläuferprotein nach Ausführungsform 7, wobei das SU-Element einen "erhöhten Anteil" an geladenen, d.h. (z.B. bei pH=7) eine Gesamtladung von ungleich 0, wie z.B. +20 bis -20. oder +10 bis -10 oder +5 bis -5, insbesondere negativ geladenen Aminosäureresten aufweist, wie z.B. bei pH=7, eine Gesamtladung von ungleich 0, wie z.B. -1 bis -20 insbesondere - 4 bis -10.
9. Ein Vorläuferprotein nach Ausführungsform 8, wobei das SU-Element im Vorläuferprotein zur Ausbildung einer amphiphilen Helixstruktur befähigt ist.
10. Ein Vorläuferprotein nach Ausführungsform 9, wobei das SU-Element ein amphiphiles Peptid ist, umfassend einen Sequenzabschnitt von wenigstens sieben peptidisch verknüpften Aminosäuren, die zur Ausbildung einer amphiphilen alpha-Helix befähigt sind, wobei die Helix in ihrer vertikalen Projektion eine Trennung der Aminosäurereste in eine hydrophobe und eine hydrophile Helixhälfte aufweist, die hydrophobe Helixhälfte mindestens 3, wie z.B. 3 oder 4 in der vertikalen Projektion, benachbarte gleiche oder verschiedene hydrophobe Aminosäurereste aufweist und die hydrophile Helixhälfte mindestens 3, wie z.B. 3 oder 4 in der vertikalen Projektion benachbarte gleiche oder verschiedene hydrophile Aminosäurereste aufweist.
11. Ein Vorläuferprotein nach Ausführungsform 8, 9 oder 10, wobei der Anteil an geladenen Aminosäureresten des SU-Elements so gewählt ist, dass die Gesamtnettoladung des Vorläuferproteins bei pH=7 größer als -10 und kleiner als +10z. B. größer als -8 und kleiner als +8; größer als -5und kleiner als +5, oder größer als - 2 und kleiner als +2 ist.
12. Vorläuferprotein nach einer der Ausführungsformen 8 bis 11, wobei das SU-Element eine Aminosäuresequenz, ausgewählt unter den Aminosäuresequenzen SEQ ID NO: 16 bis SEQ ID NO:19 und SEQ ID NO: 68 umfasst.
13. Ein Vorläuferprotein nach einer der Ausführungsformen 1 und 3 bis 12, wobei das Pep-Element eine kationische positive Gesamtladung aufweisende antimikrobielle Peptidsequenz umfasst.
14. Ein Vorläuferprotein nach Ausführungsform 13, wobei das Pep-Element eine Aminosäuresequenz, ausgewählt unter den kationischen Aminosäuresequenzen SEQ ID NO: 6 bis SEQ ID NO:15, SEQ ID NO: 23, SEQ ID NO: 26 und SEQ ID NO: 69 bis SEQ ID NO: 72 bzw. eine der unten angegebenen C-terminal und/oder N-terminal abgewandelten Formen davon umfasst.
15. Ein Vorläuferprotein nach einer der Ausführungsformen 1 oder 3 bis 6, wobei das Pep-Peptid eine Aminosäuresequenz, ausgewählt unter den Aminosäuresequenzen SEQ ID NO: 20 oder SEQ ID NO: 29 bis 67 bzw. eine der unten angegebenen C-terminal und/oder N-terminal abgewandelten Formen davon umfasst.
16. Ein Vorläuferprotein nach einer der vorhergehenden Ausführungsformen, wobei die Aux-Elemente unabhängig voneinander eine der folgenden Bedeutungen besitzen:
   SA,
   SA-SU,
   SU-SA,
   SA-SU-SA,
   SU-SA-SU,
   worin die Elemente SA und SU miteinander peptidisch verknüpft sind und die Aux-Elemente terminal mit wenigstens einem Pep-Element peptidisch, d.h. direkt oder über eine spaltbare Peptidsequenz, verknüpft sind, wobei zumindest die peptidische Verknüpfung zu den Pep-Elementen chemisch oder enzymatisch spezifisch spaltbar ist.
17. Eine Nukleinsäuresequenz kodierend für wenigstens ein Vorläuferprotein nach einer der vorhergehenden Ausführungsformen.
18. Eine Nukleinsäuresequenz nach Ausführungsform 17, umfassend wenigstens eine kodierende Sequenz gemäß SEQ ID NO: 21, 24; 27; 74 und 76.
19. Eine Expressionskassette umfassend wenigstens eine Nukleinsäuresequenz nach Ausführungsform 17 oder 18, operativ verknüpft mit wenigstens einer regulatorischen Nukleinsäuresequenz.
20. Einen rekombinanten Vektor zur Transformation eines eukaryontischen oder prokaryontischen Wirts, umfassend eine Nukleinsäuresequenz gemäß einer der Ausführungsformen 17 oder 18, oder eine Expressionskassette gemäß Ausführungsform 19.
21. Ein Verfahren zur Herstellung eines gewünschten Peptids (Pep), wobei man
   a) ein Vorläuferprotein herstellt,
      umfassend eine spaltbare repetitive Abfolge von gewünschten Peptid (Pep)-Elementen und Hilfspeptid (Aux)-Elementen, der allgemeinen Formel
      (Pep-Aux)ₓ oder
      (Aux-Pep)ₓ
      worin x >1 ist, wobei
      die Aux-Elemente gleich oder verschieden sind und Aminosäuresequenzelemente umfassen, die dem Vorläuferprotein selbstassemblierende Eigenschaften verleihen, wobei das Aux-Element ein selbstassemblierendes Peptid ((SA)-Element) umfasst
      wobei das SA-Element wenigstens eines der folgenden Sequenzmotive enthält:
      Aₙ (Motiv 1)
      (GA)ₘ (Motiv 2)
      Vₙ (Motiv 3)
      (VA)ₘ (Motiv 4)
      (WAA)ₒ (Motiv 5)
      worin A für Alanin, G für Glycin, V für Valin, n für einen ganzzahligen Wert von 8 bis 12, m für einen ganzzahligen Wert von 4 bis 10, und o für einen ganzzahligen Wert von 2 bis 6 steht; und
      die Pep-Elemente gleich oder verschieden sind und die Aminosäuresequenz gleicher oder verschiedener Peptidmoleküle mit einer Sequenzlänge von 5-70 Aminosäureresten umfassen, und die Pep-Elemente von Spaltsequenzen flankiert sind, die eine spezifische Herausspaltung der Pep-Elemente aus dem Vorläuferprotein ermöglichen, wobei die Elemente Pep und Aux miteinander peptidisch verknüpft sind und die peptidische Verknüpfung chemisch spezifisch spaltbar ist;
   b) die Pep-Peptide aus dem Vorläuferprotein abspaltet; und
   c) gegebenenfalls das Peptid enzymatisch oder chemisch modifiziert, wie z.B. amidiert, verestert, oxidiert, alkyliert oder (z.B. über native chemische Ligation oder über eine Michael Addition) mit einem weiteren Molekül verknüpft; wobei z.B. das Peptid mit einem Molekül modifiziert wird, das die Hydrophobizität des Peptids erhöht, wie z.B. mit Molekül modifiziert wird, das einen Alkylrest enthält; wobei die Modifizierung vor oder nach einer optionalen Aufreinigung des Peptids erfolgen kann, wie dies auch durch die beiliegenden Beispiele weiter veranschaulicht wird.

   Geeignete Alkylreste sind z.B. C₂-C₁₆-Alkylreste, wie Ethyl, i- oder n-Propyl, n-, i-, sec.- oder tert.-Butyl, n- oder i-Pentyl; außerdem n-Hexyl, n-Heptyl, n-Octyl, n-Nonyl, n-Decyl, n-Undecyl, n-Dodecyl n-Tridecyl, n-Tetradecyl, n-Pentadecyl und n-Hexadecyl, sowie die ein- oder mehrfach verzweigten Analoga davon, sowie gegebenenfalls substituierte Modifikationen davon, welche ein oder mehrere, wie z.B. 1, 2 oder 3 Halogen- (wie z.B. F, Cl, Br), Hydroxy-, Mercapto- Amino, C₁-C₄-Alkylamino- Substituenten tragen, oder durch ein oder mehrere, wie z.B. 1, 2 oder 3 Heteroatome, wie O oder N, in der Alkylkette unterbrochen sein können. C₁-C₄-Alkyl seht insbesondere für Methyl, Ethyl, i- oder n-Propyl, n-, i-, sec.- oder tert.-Butyl.
22. Ein Verfahren nach Ausführungsform 21, wobei man das Vorläuferprotein in einem rekombinanten Mikroorganismus produziert, der wenigstens einen Vektor nach Ausführungsform 20 trägt.
23. Ein Verfahren nach Ausführungsform 22, wobei man das Vorläuferprotein in einem rekombinanten *E. coli*-Stamm produziert.
24. Ein Verfahren nach einer der Ausführungsformen 21 bis 23, wobei man das exprimierte Vorläuferprotein, nachdem man es gegebenenfalls in eine stabil assoziierte Form überführt hat, reinigt und zur Freisetzung des gewünschten Peptids (Pep) chemisch oder enzymatisch spaltet.
25. Ein Verfahren nach einer der Ausführungsformen 21 bis 23, wobei ein Vorläuferprotein gemäß Ausführungsform 13 oder 14, wie z.B. ein Vorläuferprotein enthaltend P18-Peptidbausteine gemäß SEQ ID NO:23 oder SEQ ID NO:6, produziert wird.
26. Ein Verfahren nach Ausführungsform 25, welches folgende Aufarbeitungsschritte beinhaltet:
   - Waschen der Assoziate des Vorläuferproteins mit einem Lösungsmittel, das verunreinigende Proteine, jedoch die Assoziate nicht oder im Wesentlichen nicht löst, wie z.B. 0,1 M bis 1,0 M NaOH.
   - Spaltung der Vorläuferproteine, z.B. mit einer Säure, wenn das gewünschte Peptid, wie z.B. P18, über säurespaltbare Gruppen in das Vorläuferprotein eingebunden ist.
27. Ein Verfahren nach Ausführungsform 26, welches mindestens einen der folgenden zusätzlichen Aufarbeitungsschritte beinhaltet:
   - Behandlung der Assoziate des Vorläuferproteins nach Zellaufschluss mit einem Fällungshilfsmittel wie z.B. Phosphorsäure
   - Aufreinigung des Peptids aus dem Spaltansatz mit einem chromatographischen Verfahren;
   - Waschen des aufgereinigten und getrockneten Peptids mit einem sauren Lösungsmittel oder Lösungsmittelgemisch.
28. Ein Verfahren nach einer der Ausführungsformen 20 bis 27 zur Herstellung des Peptids gemäß SEQ ID NO:23, welches folgende Aufarbeitungsschritte beinhaltet:
   - Behandlung der Assoziate des Vorläuferproteins nach Zellaufschluß durch Zugabe von 85%iger Phosphorsäure bis pH = 3 erreicht ist
   - Waschen der Assoziate des Vorläuferproteins mit einer Natriumhydroxidlösung, wie z.B. 0,4 M NaOH
   - Spalten des Vorläuferproteins mit Phosphorsäure oder Ameisensäure, wie z.B. 2% Phosphorsäure
   - gegebenenfalls Waschen des getrockneten Peptids mit Hexansäure oder einem Gemisch aus 99 Teilen Hexan und einem Teil Essigsäure
28. Ein Verfahren nach einer der Ausführungsformen 20 bis 27 zur Herstellung des Peptids gemäß SEQ ID NO:6 welches folgende Aufarbeitungsschritte beinhaltet:
   - Hydrolysieren bzw. Spalten der Pellets, z.B. mittels 5 % iger H₃PO₄;
   - Zentrifugation;
   - Einstellen des pH-Werts des Überstands auf etwa 4,0, z.B. mit 25% NaOH
   - Aufreinigen des Überstands durch Kationenaustauschchromatographie
   - Fällung des gewünschten Peptids z.B. durch Zugabe von NaOH zum Eluat
   - Zentrifugation;
   - Resuspension des Pellets in Wasser
   - Lösen des Peptids, z.B. durch Zugabe von Essigsäure
   - Lyophilisierung
29. Ein Gegenstand der Offenbarung betrifft das P18 Peptid (SEQ ID NO:23) sowie das Peptid SEQ ID NO:6 und dessen erfindungsgemäße Herstellung, sowie dessen Verwendung in kosmetrischen oder pharmazeutischen Mitteln zur Behandlung oder Prävention von Schuppen, insbesondere Kopfschuppen; oder zur Hemmung des Wachstums und/oder der Aktivität von lipophilen Pilzen, insbesondere *Malassezia ssp., wie insbesondere Malassezia furfur.* Dies wird beispielsweise auch beschrieben in der älteren internationalen Patentanmeldung PCT/EP2008/010912, Anmeldetag 19. Dezember 2008, auf deren Offenbarung hiermit ausdrücklich Bezug genommen wird.
30. Verfahren nach Ausführungsform 21 oder 22, wobei das Vorläuferprotein stabile Assoziate bildet, die durch 0,2 M NaOH innerhalb einer Stunde oder von 2 M Harnstoff oder 1 M Guanidiumhydrochlorid innerhalb von 10 min bei Raumtemperatur nicht auflösbar sind.
31. Verfahren nach einer der Ausführungsformen 21, 22 oder 30, wobei das Vorläuferprotein wie in einer der Ausführungsformen 6 bis 16 definiert ist, oder von einer Nukleinsäuresequenz nach einer der Ausführungsformen 17 und 18 kodiert wird.
Weiterhin sind hierein folgende nichterfindungsgemäße Ausführungsformen beschrieben:
32. Ein Vorläuferprotein umfassend eine spaltbare Abfolge von gewünschten Peptid (Pep)-Elementen und Hilfspeptid (Aux')-Elementen, der allgemeinen Formel
   (Pep-Aux')x oder
   (Aux'-Pep)x
   worin x >1 ist, wobei
   die Aux'-Elemente gleich oder verschieden sind, und ein amphiphiles alpha-Helix-bildendes Peptid umfassen, wobei das amphiphile Peptid einen Sequenzabschnitt von wenigstens sieben peptidisch verknüpften Aminosäuren umfasst, die zur Ausbildung einer amphiphilen alpha-Helix befähigt sind, wobei die Helix in ihrer vertikalen Projektion eine Trennung der Aminosäurereste in eine hydrophobe und eine hydrophile Helixhälfte aufweist, die hydrophobe Helixhälfte mindestens 3 wie z.B. 3 oder 4 in der vertikalen Projektion benachbarte gleiche oder verschiedene hydrophobe Aminosäurereste aufweist und die hydrophile Helixhälfte mindestens 3 wie z.B. 3 oder 4 in der vertikalen Projektion benachbarte gleiche oder ver-schiedene hydrophile Aminosäurereste aufweist; und
   die Pep-Elemente gleich oder verschieden sind und die Aminosäuresequenz gleicher oder verschiedener Peptidmoleküle umfassen.
32. Ein Vorläuferprotein nach Ausführungsform 31, wobei die Aux'-Elemente wenigstens ein selbstassemblierendes Peptid (SA)-Element gemäß der Definition in einer der Ausführungsformen 4 und 5 umfasst.
33. Vorläuferprotein nach Ausführungsform 31 oder 32, wobei das gewünschte Peptid (Pep) ein kationisches antimikrobielles Peptid ist und das Aux'-Element ein anionisches, eine amphiphile alpha-Helix ausbildendes Peptid ist.
34. Verwendung eines amphiphilen Peptids als Schutzpeptid zur rekombinanten Herstellung eines davon verschiedenen antimikrobiellen gewünschten Peptids; wobei das amphiphile Peptid einen Sequenzabschnitt von wenigstens sieben peptidisch verknüpften Aminosäuren umfasst, die zur Ausbildung einer amphiphilen alpha-Helix befähigt sind, wobei die Helix in ihrer vertikalen Projektion eine Trennung der Aminosäurereste in eine hydrophobe und eine hydrophile Helixhälfte aufweist, die hydrophobe Helixhälfte mindestens 3 (in der vertikalen Projektion) benachbarte gleiche oder verschiedene hydrophobe Aminosäurereste aufweist und die hydrophile Helixhälfte mindestens 3 (in der vertikalen Projektion) benachbarte gleiche oder verschiedene hydrophile Aminosäurereste aufweist.
35. Verwendung nach Ausführungsform 34, wobei das gewünschte Peptid (Pep) ein kationisches antimikrobielles Peptid ist und das Aux'-Element ein anionisches, eine amphiphile alpha-Helix ausbildendes Peptid ist.

### Detaillierte Beschreibung einzelner Aspekte der Erfindung

### 1. Peptide

Peptide (Pep) gemäß der vorliegenden Erfindung, auch als "gewünschte Peptide" oder "Target-Petide" bezeichenbar, sind Aminosäureketten, bei denen 5 bis 70 und insbesondere 7 bis 50, wie z.B. 10 bis 40, 12 bis 35 oder 15 bis 25, Aminosäuren über Peptidbindungen verknüpft sind. Peptide können aus beliebigen α-Aminosäuren, insbesondere aus den proteinogenen Aminosäuren, aufgebaut sein.

Die Peptide können bestimmte gewünschte biologische oder chemische sowie insbesondere auch pharmakologisch einsetzbare Eigenschaften besitzen. Beispiele für solche Eigenschaften sind: antimikrobielle Aktivität, spezifische Bindung an bestimmte Oberflächen, keimbildende Eigenschaften bei Kristallisationsprozessen und Partikelbildung, Steuerung von Kristallstrukturen, Bindung von Metallen oder Metallionen, oberflächenaktive Eigenschaften, emulgierende Eigenschaften, schaumstabilisierende Eigenschaften, Beeinflussung von zellulärer Adsorption.

Die Peptide können eine oder mehrere dieser Eigenschaften aufweisen.

Ebenfalls ist hierin beschrieben eine Methode zur Herstellung antimikrobieller Peptide. Solche "antimikrobiellen Peptide" sind dadurch gekennzeichnet, dass in Anwesenheit von Konzentrationen ≤100 µM des antimikrobiellen Peptids das Wachstum und/oder die Vervielfältigung wenigstens einer Art von grampositiven oder gramnegativen Bakterien und/oder wenigstens einer Art von Hefen und/oder wenigstens einer Art von filamentösen Pilzen und/oder wenigstens einer Art von Algen inhibiert wird und/oder die Zellen des entsprechenden Organismus abgetötet werden.

Ebenfalls ist hierin beschrieben die Bereitstellung kationischer antimikrobieller Peptide. Kationische antimikrobielle Peptide sind dadurch gekennzeichnet, dass sie eine antimikrobielle Wirkung gemäß der oben aufgeführten Definition sowie eine Nettoladung bei pH 7 größer als 0 aufweisen.

Derartige kationische Peptide enthalten z.B. die folgende Sequenz:
X₁ X₂K X₃ X₄ X₅KIP X₁₀ KFX₆X₇ X₈ AX₉KF (SEQ ID NO: 7)
worin
X₁₀ für eine Peptidbindung oder ein oder zwei beliebige basische oder hydrophobe Aminosäurereste oder ein oder zwei Prolinreste steht und
X₁ bis X₉ beliebige basische oder von Prolin verschiedene hydrophobe Aminosäurereste bedeuten;
und/oder Mutanten oder Derivate davon;
wobei die im Vorläuferprotein enthaltenen repetitiven Sequenzmotive gleich oder verschieden sein können.

In einer weiteren besonderen Ausführungsform betrifft die Erfindung die Herstellung von Peptiden, die die folgende Sequenz enthalten:
X₁ X₂K X₃ X₄ X₅KIP X₁₁ X₁₂ KFX₆X₇ X₈ AX₉KF (SEQ ID NO: 8)
worin
X₁ für Lysin, Arginin oder Phenylalanin,
X₂ für Lysin oder Tryptophan,
X₃ für Leucin oder Lysin,
X₄ für Phenylalanin oder Leucin,
X₅ für Leucin oder Lysin,
X₆ für Leucin oder Lysin,
X₇ für Histidin oder Lysin,
X₈ für Alanin, Leucin, Valin oder Serin,
X₉ für Leucin oder Lysin,
X₁₁ für Prolin oder eine chemische Bindung, und
X₁₂ für Prolin oder eine chemische Bindung steht,
und/oder Mutanten oder Derivate davon;
wobei die im Vorläuferprotein enthaltenen repetitiven Sequenzmotive gleich oder verschieden sind;
Nicht limitierende Beispiele obiger Sequenzen oder repetitiver Sequenzmotive sind SEQ ID NO:6, SEQ ID NO:9 - SEQ ID NO:15, SEQ ID NO:23, SEQ ID NO:69, SEQ ID NO:71, und/oder eine Mutanten oder Derivate davon.

Weiter geeignete Peptide sind z.B. beschreiben in der Internationalen Patentanmeldung der vorliegenden Patentanmelderin, PCT/EP2008/010912, Anmeldetag 19.12.2008, worauf hiermit ausdrücklich Bezug genommen wird.

### 2. Repetitive Vorläuferproteine

Repetitive Vorläuferproteine gemäß der vorliegenden Erfindung sind dadurch gekennzeichnet, dass mindestens 60%, insbesondere mindestens 80% ihrer Aminosäuresequenz, wie z.B. 60- 99%, 70-95%, 75-85%, jeweils bezogen auf die Gesamtsequenzlänge, aus peptidischen Wiederholungseinheiten (wie unten definiert) bestehen. Der übrige Anteil kann z.B. nicht-repititive Peptide, wie z.B. Signalpeptide, Tags und dergleichen umfassen.

### 3. Wiederholungseinheiten

Peptidische Wiederholungseinheiten enthalten wenigstens ein Peptid, welches gemäß der vorliegenden Erfindung in vorteilhafter Weise hergestellt wird, und sind prinzipiell wie folgt aufgebaut
(Pep-Aux)ₓ oder
(Aux-Pep)ₓ
worin x >1 ist, und wobei Pep für das oben bezeichnete Peptid steht und Aux wie in Anspruch 1 definiert ist.

Eine Wiederholungseinheit (Pep-Aux, bzw. Aux-Pep) gemäß der vorliegenden Erfindung ist eine Aminosäuresequenz mit einer Länge von 10-200, wie z.B. 20-130 und oder 30-80 Aminosäuren, die innerhalb eines Vorläuferproteins mehrere Mal als identische Sequenz oder als Variation einer bestimmten Sequenz mit mindestens 70%, wie z.B. mindestens 80% und insbesondere mindestens etwa 90% Identität, wie z.B. 91, 92, 93, 94, 95, 96, 97, 98 oder 99% Identität, enthalten ist. Repetitive Vorläuferproteine gemäß der vorliegenden Erfindung können somit z.B. identische Kopien oder Variationen einer einzigen oder mehrerer unterschiedlicher Aminosäuresequenzen, wie z.B. des Pep und/oder des Aux Bausteins, enthalten.

In einem repetitiven Vorläuferprotein kann außerdem eine beliebige Anzahl von obigen Wiederholungseinheiten, wie z.B. >1-100, >1-50, oder 2-32 und insbesondere 4-16, aneinandergefügt sein.

Der Anteil des erfindungsgemäßen Peptids an der Wiederholungseinheit bezogen auf die Molmasse beträgt 20%-80%, wie z.B.30%-70% oder 40 bis 60%. Der übrigen Anteil der Wiederholungseinheit wird von den Aux-Sequenzen eingenommen, insbesondere den oben definierten SA und SU Sequenzen, sowie ggf. spezifischen Spaltsequenzen, zur selektiven Entfernung des Pep-Bausteins.

### 4. Hilfssequenzen

Hilfssequenzen im weitesten Sinne sind Aminosäuresequenzen in einem erfindungsgemäßen Vorläuferprotein, die die Eigenschaften des Vorläuferproteins so beeinflussen, dass die Expression, die Stabilität und/oder die Aufarbeitung des Vorläuferproteins verbessert wird. Hilfssequenzen können in einem repetitiven Vorläuferprotein Teil einer Wiederholungseinheit sein (die oben bezeichneten Aux-Bausteine) oder aminoterminal oder carboxyterminal an das Vorläuferprotein angefügt sein, wie z.B. 6 x His-Tag (HHHHHH), T7-Tag (MASMTGGQQMG), S-Tag (KETAAAKFERQHMDS), c-Myc-Tag (EQKLISEEDL), Strep-Tag (WSHPQFEK) oder HA-Tag (YPYDVPDYA), Glutathione S-Transferase, Maltose-Bindeprotein, Cellulose Bindeprotein. Diese und weitere Hilfssequenzen sind in Terpe; Appl Microbiol Biotechnol; 60(5): 523-33 (2003) beschrieben. Des Weiteren eignen sich die Hilfsequenzen CanA (Mai,"In Vitro Untersuchungen zum extrazellulären Netzwerk von Pyrodictium abyssi TAG11" Dissertation, Universität Regensburg (1998)) und yaaD (Wohlleben Eur Biophys J, (2009) online publication) dazu, aminoterminal oder carboxyterminal an das Vorläuferprotein angefügt zu werden.

In einer Ausführungsform enthält das Vorläuferprotein Hilfssequenzen, die die Löslichkeit des Vorläuferproteins beeinflussen.

Die Hilfssequenzen verleihen dem Vorläuferprotein "selbstassemblierende" Eigenschaften. Die selbstassemblierenden Eigenschaften des Vorläuferproteins sind dadurch gekennzeichnet, dass das Vorläuferprotein bereits während der Expression "spontan", d.h. von selbst ohne zusätzlich erforderliche Maßnahme, stabile Assoziate bildet, oder die Bildung solcher stabiler Assoziate aus löslichen Vorläuferproteinen "induzierbar", d.h. durch einen Trigger, ausgelöst werden kann. Vorläuferproteine, die selbstassemblierende Eigenschaften aufweisen, haben gegenüber anderen Vorläuferproteinen den Vorteil, dass sie auf einfache und effiziente Art und Weise aufgereinigt werden können. Derartige Assoziate umfassen, in der Regel ausschließlich oder im Wesentlichen, die Ausbildung nicht-kovalenter Bindungen, wie z.B. Wasserstoffbrücken, ionischer und/oder hydrophober Wechselwirkungen.

Selbstassemblierende Sequenzen weisen eine Länge von mindestens 8 zusammenhängenden Aminosäuren auf. Geeignete Sequenzen sind beispielsweise in an sich bekannten Protein lokalisierbar, für welche die Assemblierung zu höhermolekularen Assoziaten schon nachgewiesen ist. Beispiele für solche Assoziate sind amyloide Fibrillen, Aktin- oder Myosinfilamente, Proteinfasern wie Elastinfasern, Kollagenfasern, Muschelbyssusfäden, Keratinfasern, oder Seidenfäden. Diese und weitere Proteine, die selbstassemblierende Sequenzen enthalten sind in Scheibel, Current Opinion in Biotechnology 16; 1-7 (2005) beschrieben, worauf hiermit ausdrücklich Bezug genommen wird.

Als "Trigger" können Lösungen von kosmotropen Salzen eingesetzt werden. Beispielhaft sind kosmotrope Salze zu nennen, die mindestens eine Ionenart enthalten, die entsprechend der sogenannten Hofmeister-Reihe stärker kosmotrope Eigenschaften aufweist, als Natrium- oder Chloridionen. Beispiele für solche Salze sind Kaliumphosphat und Ammoniumsulfat. Beispiele für solche Salzlösungen sind 0,5 M Kaliumphosphat oder 0,8 M Ammoniumsulfat.

Stabile erfindungsgemäße Assoziate von Vorläuferproteinen sind dadurch gekennzeichnet, dass sie während der Behandlung mit Lösungen, die typischerweise eine Vielzahl von aggregierten Proteinen solubilisieren können, ihre assoziierte Form für eine bestimmte Zeit beibehalten und auf diese Art von Proteinverunreinigungen getrennt werden können. Beispiele für solche Lösungen sind Lösungen von Basen, Säuren, Harnstoff, Salzen und Detergenzien. Insbesondere sind die erfindungsgemäßen stabilen Assoziate eine bestimmte Zeit unlöslich in Lösungen von Alkalihydroxiden, Harnstoff, Guanidiniumsalzen oder geladenen Detergenzien, wie z.B. Alkyltrimethylammoniumsalze oder Alkylsulfate.

Insbesondere sind die stabilen Assoziate eine bestimmte Zeit unlöslich in Lösungen von ≥ 0,2 M Natriumhydroxid, ≥ 2 M Harnstoff, ≥ 1M Guanidiniumhydrochlorid, ≥ 1M Guanidiniumthiocyanat oder ≥ 0,1%Natriumdodecylsulfat oder ≥ 0,1% Cetyltrimethylammoniumbromid. Insbesondere sind stabile Assoziate von Vorläuferproteinen in den genannten Lösungen stabil für ≥ 10 min, wie z.B. ≥ 30 min und insbesondere ≥ 60 min.

Ein stabiles Assoziat ist insbesondere dann gegeben, wenn es
a) durch 0,2 M NaOH innerhalb einer Stunde und/oder
b) von 2 M Harnstoff und/oder
c) von1 M Guanidiniumhydrochlorid
innerhalb von 10 min bei Raumtemperatur (i.e. etwa 20 °C) nicht auflösbar ist.

In einer weiteren besonderen Ausführungsform enthält das Vorläuferprotein Hilfssequenzen (SU), die die Wirtszelle vor schädigenden Einflüssen des repetitiven Vorläuferproteins schützt.

In einer speziellen Ausführungsform enthält das Vorläuferprotein Hilfssequenzen SU, die die Wirtszelle vor schädigenden Einflüssen von kationischen antimikrobiellen Peptidsequenzen schützt, die in dem repetitiven Vorläuferprotein enthalten sind. Insbesondere enthalten diese Schutzsequenzen negativ geladene Aminosäuren (Asp, Glu). Insbesondere enthält die Hilfssequenz eine Anzahl von negativ geladenen Aminosäuren Glutamat und / oder Aspartat, so dass die Gesamtnettoladung bei pH=7 innerhalb des repetitiven Vorläuferproteins größer als -10 und kleiner als +10, vor allem größer als -5 und kleiner als +5, wie z.B. größer als -2 und kleiner als +2 ist.

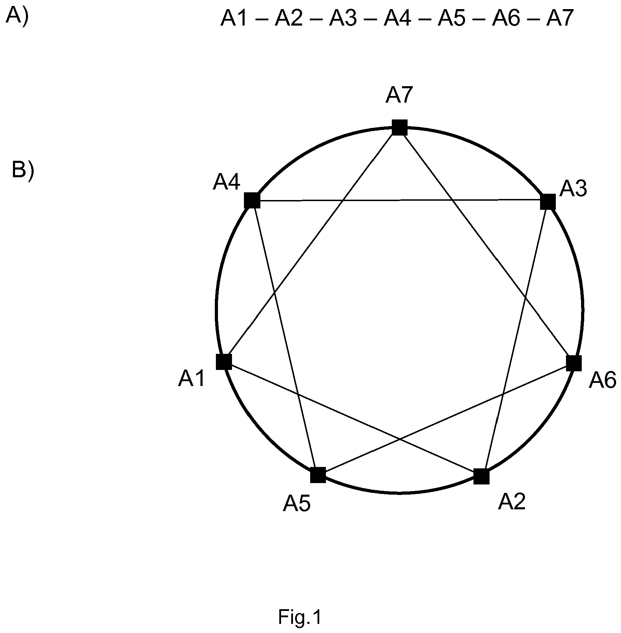

In einer weiteren speziellen Ausführungsform bildet die negativ geladene Schutzsequenz eine amphipathische Helix aus. Eine amphipathische Helix gemäß der vorliegenden Erfindung wird dann gebildet, wenn bei der kreisförmigen Anordnung (d.h. in seiner axialen (entlang der Helix Achse) Projektion bzw. Draufsicht) einer Sequenz von 7 in der Primärstruktur aufeinanderfolgende Aminosäuren (A1 - A7) in der folgenden Reihenfolge: A1 - A5 - A2 - A6 - A3 - A7 - A4 (Figur 1) mindestens 3 auf dem Kreis nebeneinander liegende Aminosäuren hydrophobe Aminosäuren (Ala, Met, Cys, Phe, Leu, Val, Ile) oder Glycin sind und 3 auf dem Kreis nebeneinander liegende Aminosäuren hydrophile Aminosäuren (Thr, Ser, Trp, Tyr, Pro, His, Glu, Gln, Asp, Asn, Lys, Arg) oder Glycin sind. Diese kreisförmige Anordnung wird auch als "helical wheel projection" bezeichnet.

In einer bevorzugten Ausführungsform entspricht die negativ geladene Schutzsequenz einer der Sequenzen SEQ ID NO: 16 - SEQ ID NO: 19

### 5. Spaltsequenzen

Spaltsequenzen sind Aminosäuresequenzen, die vor und nach den erfindungsgemäß gewünschten Peptidsequenzen (Pep) angeordnet sind. Diese Sequenzen ermöglichen die "spezifische" Herausspaltung der Pep-Bausteine aus dem repetitiven Vorläuferprotein. "Spezifisch" bedeutet in diesem Zusammenhang, das die Spaltung im Vorläuferprotein, im Wesentlichen, insbesondere ausschließlich an einer oder mehreren definierten Positionen erfolgt, wodurch das gewünschte Peptid oder eine Vorstufe davon, abgetrennt wird.

Eine "Vorstufe" kann z.B. darin bestehen, dass an einem oder beiden Enden der peptidkette Aminosäurereste enthalten sind, welche nicht Bestandteil der nativen, ursprünglichen Peptidsequenz sind, dessen Weiterverwendung und Funktionalität aber nicht stören, oder erforderlichenfalls mit herkömmlichen chemischen oder biochemischen Methoden abspaltbar sind.

Spaltsequenzen können als spezifische Erkennungssequenz für proteolytisch aktive Enzyme fungieren, die an diese Sequenz binden und zwischen zwei bestimmten Aminosäuren die Peptidbindung spalten. Beispiele sind Erkennungssequenzen für Arg-C proteinase, Asp-N-Endopeptidase, Caspasen, Chymotrypsin, Clostripain, Enterokinase, Factor Xa, Glutamylendopeptidase, Granzyme B, LysC Lysylendopeptidase (Achromobacterproteinase I) LysN Peptidyl-Lys metalloendopeptidase, Pepsin, Proline Endopeptidase, Proteinase K, Staphylococcal peptidase I, Thermolysin, Thrombin, Trypsin. Die entsprechenden Erkennungssequenzen sind in der Literatur beschrieben z.B. in Keil, "Specificity of proteolysis" p. 335 Springer-Verlag (1992).

Alternativ ermöglichen bestimmte Aminosäuresequenzen die selektive Spaltung des Polypeptidrückgrades mit bestimmten Chemikalien, wie beispielsweise BNPS-Skatole (2-(2'-Nitrophenylsulfenyl)-3-methyl-3-bromoinolenine), Bromcyan, Säuren, Hydroxylamin, Iodosobenzoesäure, NTCB (2-nitro-5-thiocyanobenzoesäure).

Insbesondere ermöglichen die verwendeten Spaltsequenzen die Spaltung der repetitiven Vorläuferproteine mit Chemikalien. Besonders geeignete Spaltsequenzen umfassen die Sequenzmotive Asn-Gly die eine Spaltung mit Hydroxylamin oder Asp-Pro oder Asp-Xxx, die eine Spaltung mit Säure erlaubt, wobei Xxx eine beliebige proteinogene Aminosäure darstellt.

### 6. Weitere Ausgestaltungen erfindungsgemäßer Sequenzen

### 6.1 Aminosäuresequenzen

Neben den hierin konkret offenbarten Sequenzen für Peptide (Pep), und Hilfssequenzen (Aux, SA, SU), repetitive Sequenzen, Spaltsequenzen und Sequenzen für repetitive Vorläuferproteine sind auch funktionale Äquivalente, funktionale Derivate und Salze dieser Sequenz Gegenstand der Erfindung.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen, aber trotzdem die gleichen Eigenschaften der ursprünglich unveränderten Peptide besitzen. "Funktionale Äquivalente" umfassen somit die durch eine oder mehrere Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen.

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

Unter dem Ausdruck "Salze" versteht man sowohl Salze von Carboxylgruppen als auch Säureadditionssalze von Aminogruppen der erfindungsgemäßen Peptidmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" (oder "Derivate") erfindungsgemäßer Polypeptide können an funktionellen Aminosäure-Seitengruppen oder an deren N- oder C-terminalem Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen. Weiterhin können N- und/oder C-terminal zusätzlich 1 bis 5, wie z. B. 2, 3 oder 4 beliebige D- oder L-Aminosäurereste kovalent (peptidisch) gebunden sein.

### 6.2 Nukleinsäuren, Expressionskonstrukte, Vektoren und diese enthaltende Mikroorganismen

### Nukleinsäuren:

Die Erfindung umfasst weiterhin die für die erfindungsgemäß eingesetzten Peptid und Proteinsequenzen kodierenden Nukleinsäuremoleküle.

Alle hierin erwähnten Nukleinsäuresequenzen (einzel- und doppelsträngige DNA- und RNA-Sequenzen, wie z. B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Die Erfindung betrifft isolierte Nukleinsäuremoleküle, welche für erfindungsgemäße Polypeptide bzw. Proteine oder biologisch aktive Abschnitte davon kodieren. Offenbart sind hierin auch Nukleinsäurefragmente, die z. B. als Hybridisierungssonden oder Primer zur Identifizierung oder Amplifizierung von erfindungsgemäßen kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten.

Ein "isoliertes" Nukleinsäuremolekül wird von anderen Nukleinsäuremolekülen abgetrennt, die in der natürlichen Quelle der Nukleinsäure zugegen sind, und kann überdies im Wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standardtechniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z. B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies läßt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z. B. mit einem automatischen DNA-Synthesegerät, hergestellt werden.

Die Offenbarung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z. B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Erfindungsgemäß umfasst sind auch solche Nukleinsäuresequenzen, die sogenannte stumme Mutationen umfassen oder entsprechend der Codon-Nutzung eines speziellen Ursprungs- oder Wirtsorganismus im Vergleich zu einer konkret genannten Sequenz verändert sind, ebenso wie natürlich vorkommende Varianten, wie z. B. Spleißvarianten oder Allelvarianten davon. Offenbart sind ebenso durch konservative Nukleotidsubstutionen (d. h. die betreffende Aminosäure wird durch eine Aminosäure gleicher Ladung, Größe, Polarität und/oder Löslichkeit ersetzt) erhältliche Sequenzen.

Gegenstand der Offenbarung sind auch die durch Sequenzpolymorphismen von den konkret offenbarten Nukleinsäuren abgeleiteten Moleküle. Diese genetischen Polymorphismen können zwischen Individuen innerhalb einer Population auf Grund der natürlichen Variation existieren. Diese natürlichen Variationen bewirken üblicherweise eine Varianz von 1 bis 5 % in der Nukleotidsequenz eines Gens.

Weiterhin umfasst die Offenbarung auch Nukleinsäuresequenzen, welchen mit oben genannten kodierenden Sequenzen hybridisieren oder dazu komplementär sind. Diese Polynukleotide lassen sich bei Durchmusterung von genomischen oder cDNA-Banken auffinden und gegebenenfalls daraus mit geeigneten Primern mittels PCR vermehren und anschließend beispielsweise mit geeigneten Sonden isolieren. Eine weitere Möglichkeit bietet die Transformation geeigneter Mikroorganismen mit erfindungsgemäßen Polynukleotiden oder Vektoren, die Vermehrung der Mikroorganismen und damit der Polynukleotide und deren anschließende Isolierung. Darüber hinaus können erfindungsgemäße Polynukleotide auch auf chemischem Wege synthetisiert werden. Unter der Eigenschaft, an Polynukleotide "hybridisieren" zu können, versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht komplementären Partnern unterbleiben. Dazu sollten die Sequenzen zu 70-100 %, vorzugsweise zu 90-100 %, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50-70 °C, vorzugsweise 60-65 °C warmen Waschlösung, beispielsweise 0,1 x SSC-Puffer mit 0,1 % SDS (20 x SSC: 3 M NaCl, 0,3 M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z. B. in Ausubel et al., Current Protocols in Molecular Biology, John Wiley & Sons, N.Y. (1989), 6.3.1-6.3.6, beschrieben.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

**Multiple alignment parameter:**

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

**Pairwise alignment parameter:**

| | |
|---|---|
| FAST algorithm on K-tuple size | 1 |
| Gap penalty | 3 |
| Window size | 5 |
| Number of best diagonals | 5 |

### Expressionskonstrukte und Vektoren:

Gegenstand der Erfindung sind außerdem Expressionskonstrukte, enthaltend unter der genetischen Kontrolle regulativer Nukleinsäuresequenzen eine für ein erfindungsgemäßes Vorläuferprotein kodierende Nukleinsäuresequenz, sowie Vektoren, umfassend wenigstens eines dieser Expressionskonstrukte. Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targetingsequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z. B. beschrieben in Goeddel, Gene Expression Technology: Methods in Enzymology 185, Academic Press, San Diego, CA (1990).

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für brauchbare Promotoren sind: cos-, tac-, trp-, tet-, trp-tet-, lpp-, lac-, lpplac-, laclq-, T7-, T5-, T3-, gal-, trc-, ara-, SP6-, lambda-PR- oder im lambda-PL-Promotor, die vorteilhafterweise in gramnegativen Bakterien Anwendung finden; sowie die grampositiven Promotoren amy und SPO2, die Hefepromotoren ADC1, MFa , AC, P-60, CYC1, GAPDH oder die Pflanzenpromotoren CaMV/35S, SSU, OCS, lib4, usp, STLS1, B33, not oder der Ubiquitin- oder Phaseolinpromotor. Besonders bevorzugt ist die Verwendung induzierbarer Promotoren, wie z. B. licht- und insbesondere temperaturinduzierbare Promotoren, wie der PᵣPₗ-Promotor. Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Viren, wie SV40, CMV, Baculovirus und A-denovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Beispiele für geeignete Expressionsvektoren können genannt werden:
Übliche Fusionsexpressionsvektoren, wie pGEX (Pharmacia Biotech Inc; Smith, D.B. und Johnson, K.S. (1988) Gene 67:31-40), pMAL (New England Biolabs, Beverly, MA) und pRIT 5 (Pharmacia, Piscataway, NJ), bei denen Glutathion-S-Transferase (GST), Maltose E bindendes Protein bzw. Protein A an das rekombinante Zielprotein fusioniert wird.
Nicht-Fusionsprotein-Expressionsvektoren wie pTrc (Amann et al., (1988) Gene 69:301-315) und pET 11d (Studier et al. Gene Expression Technology: Methods in Enzymology 185, Academic Press, San Diego, Kalifornien (1990) 60-89).
Hefe-Expressionsvektor zur Expression in der Hefe S. *cerevisiae* , wie pYepSec1 (Baldari et al., (1987) Embo J. 6:229-234), pMFa (Kurjan und Herskowitz (1982) Cell 30:933-943), pJRY88 (Schultz et al., (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie filamentösen Pilzen, eignen, umfassen diejenigen, die eingehend beschrieben sind in: van den Hondel, C.A.M.J.J. & Punt, P.J. (1991) "Gene transfer systems and vector development for filamentous fungi, in: Applied Molecular Genetics of Fungi, J.F. Peberdy et al., Hrsg., S. 1-28, Cambridge University Press: Cambridge.
Baculovirus-Vektoren, die zur Expression von Proteinen in gezüchteten Insektenzellen (beiispielsweise Sf9-Zellen) verfügbar sind, umfassen die pAc-Reihe (Smith et al., (1983) Mol. Cell Biol. 3:2156-2165) und die pVL-Reihe (Lucklow und Summers, (1989) Virology 170:31-39).
Pflanzen-Expressionsvektoren, wie solche, die eingehend beschrieben sind in: Becker, D., Kemper, E., Schell, J. und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721.
Säugetier-Expressionsvektoren, wie pCDM8 (Seed, B. (1987) Nature 329:840) und pMT2PC (Kaufman et al. (1987) EMBO J. 6:187-195).
Weitere geeignete Expressionssysteme für prokaryontische und eukaryotische Zellen sind in Kapitel 16 und 17 von Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989 beschrieben.

### Rekombinante Mikroorganismen:

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al., Molecular Cloning: A Laboratory Manual. 2. Aufl., Cold Spring Harbor Laboratory, Cold Spring Harbor Laboratory Press, Cold Spring Harbor, NY, 1989 beschrieben.

Erfindungsgemäß sind auch homolog rekombinierte Mikroorganismen herstellbar. Dazu wird ein Vektor hergestellt, der zumindest einen Abschnitt eines erfindungsgemäßen Gens oder einer kodierenden Sequenz enthält, worin gegebenenfalls wenigstens eine Aminosäure-Deletion, -Addition oder -Substitution eingebracht worden ist, um die erfindungsgemäße Sequenz zu verändern, z. B. funktionell zu disrumpieren ("Knockout"-Vektor). Die eingebrachte Sequenz kann z. B. auch ein Homologes aus einem Verwandten Mikroorganismus sein oder aus einer Säugetier-, Hefe- oder Insektenquelle abgeleitet sein. Der zur homologen Rekombination verwendete Vektor kann alternativ derart ausgestaltet sein, dass das endogene Gen bei homologer Rekombination mutiert oder anderweitig verändert ist, jedoch noch das funktionelle Protein kodiert (z. B. kann der stromaufwärts gelegene regulatorische Bereich derart verändert sein, dass dadurch die Expression des endogenen Proteins verändert wird). Der veränderte Abschnitt des erfindungsgemäßen Gens ist im homologen Rekombinationsvektor. Die Konstruktion geeigneter Vektoren zur homologen Rekombination ist z. B. beschrieben in Thomas, K.R. und Capecchi, M.R. (1987) Cell 51:503.

Als Wirtsorganismen sind prinzipiell alle Organismen geeignet, die eine Expression der erfindungsgemäßen Nukleinsäuren, ihrer Allelvarianten, ihrer funktionellen Äquivalente oder Derivate ermöglichen. Unter Wirtsorganismen sind beispielsweise Bakterien, Pilze, Hefen, pflanzliche oder tierische Zellen zu verstehen.

Nicht limitierende Beispiele für prokaryontische Expressionsorganismen sind *Escherichia coli, Bacillus subtilis, Bacillus megaterium, Corynebacterium glutamicum* u.a.. Nicht limitierende Beispiele für eukaryontische Expressionsorganismen sind Hefen, wie *Saccharomyces cerevisiae, Pichia pastoris* u.a., *filamentöse Pilze,* wie *Aspergillus niger, Aspergillus oryzae, Aspergillus nidulans, Trichoderma reesei, Acremonium chrysogenum* u.a., Säugetierzellen, wie Hela-Zellen, COS-Zellen, CHO-Zellen u.a., Insektenzellen, wie Sf9-Zellen, MEL-Zellen u.a., Pflanzen oder Pflanzenzellen wie *Solanum tuberosum, Nicotiana* u.a..

Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben der transformierten Zelle bewirkt. Diese können dann mittels automatischer Zellsortierung selektiert werden. Erfolgreich mit einem Vektor transformierte Mikroorganismen, die ein entsprechendes Antibiotikaresistenzgen (z. B. G418 oder Hygromycin) tragen, lassen sich durch entsprechende Antibiotika enthaltende Medien oder Nährböden selektieren. Markerproteine, die an der Zelloberfläche präsentiert werden, können zur Selektion mittels Affinitätschromatographie genutzt werden.

### 7. Rekombinante Herstellung der Vorläuferproteine und Peptide

Die erfindungsgemäß verwendeten Peptide und Vorläuferproteine sind grundsätzlich in an sich bekannter Weise rekombinant herstellbar, wobei man einen Peptid/Vorläuferprotein-produzierenden Mikroorganismus kultiviert, gegebenenfalls die Expression der Polypeptide induziert und diese aus der Kultur isoliert. Die Peptide und Vorläuferproteine können so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40 °C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989), beschrieben.

Die Zellen werden dann, falls die Peptide oder Vorläuferproteine nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z. B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Peptide oder Vorläuferproteine kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin, beschrieben.

Weiterhin können zur Isolierung des rekombinanten Peptids oder Vorläuferproteins Vektorsysteme oder Oligonukleotide verwendet werden, die die cDNA um bestimmte Nukleotidsequenzen verlängern, und damit für veränderte Polypeptide oder Fusionsproteine kodieren, die z. B. einer einfacheren Reinigung dienen. Derartige geeignete Modifikationen sind beispielsweise als Anker fungierende sogenannte "Tags", wie z. B. die als Hexa-Histidin-Anker bekannte Modifikation, oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z. B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

Insbesondere erfolgt die Herstellung der repetitiven Vorläuferproteine durch Expression synthetisch hergestellter Gensequenzen die für die erfindungsgemäßen repetitiven Vorläuferproteine kodieren. Eine Möglichkeit zur Herstellung synthetischer Gensequenzen ist in Hummerich et al. Biochemistry 43; 13604-13612 (2004) beschrieben.

Die repetitiven Vorläuferproteine können in der Wirtszelle löslich oder unlöslich vorliegen. In beiden Fällen werden die Zellen aufgeschlossen. Insbesondere erfolgt der Aufschluss mittels Hochdruckhomogenisator bei 1000 - 1500 bar. Bei löslichen repetitiven Vorläuferproteinen wird ein Großteil der zellulären Proteine durch Erhitzen des Lysats auf 60 - 100°C, wie 70 - 90°C oder 75 - 85°C ausgefällt und durch ein geeignetes Trennverfahren (z.B. Sedimentierung oder Filtration) von dem löslichen repetitiven Vorläuferprotein abgetrennt. Das repetitive Vorläuferprotein wird anschließend durch Zugabe eines kosmotropen Salzes (wie oben beschreiben) gefällt. Dabei bilden die repetitiven Vorläuferproteine stabile Assoziate. Die Endkonzentrationen der zugesetzten kosmotropen Salze können je nach Assoziat variieren und liegen etwa im Bereich von etwa 0,2 - 3 M, oder z.B. 0,8- 2 M. Optimale Konzentrationen sind in einfacher, dem Proteinchemiker geläufiger Weise zu bestimmen.

Die Assemblierung der repetitiven Vorläuferproteine kann auch ohne äußeren Trigger erfolgen. Dann assemblieren die repetitiven Vorläuferproteine bereits in der Wirtszelle zu entsprechenden stabilen Assoziaten. Die Assoziate werden nach Aufschluss der Zellen durch ein geeignetes Trennverfahren (z.B. Sedimentierung oder Filtration) von löslichen Komponenten getrennt.

Die Abtrennung der Assoziate kann durch die Zugabe von Fällungshilfsmitteln nach Aufschluss der Zellen verbessert werden. Die Fällungshilfsmittel bewirken eine weitere Verklumpung der Assoziate wodurch z.B. bei der Sedimentation geringere Beschleunigungen aufgewendet werden müssen, um die Assoziate vom wässrigen Medium zu trennen. Als Fällungshilfsmittel können Säuren, Laugen, Polymerlösungen, insbesondere wässrige Lösungen geladener Polymere verwendet werden. Beispiele für Fällungshilfsmittel sind Phosphorsäure oder Lösungen von Polyethylenimin.

Die stabilen Assoziate repetitiver Vorläuferproteine können weiter gereinigt werden. Dazu werden für diese Reinigung Lösungen verwendet, in denen die stabilen Assoziate unlöslich sind, andere Verunreinigungen dagegen gelöst werden. Insbesondere werden wässrige Lösungen von Basen, Säuren, Harnstoff, Salzen und Detergenzien verwendet. Besonders geeignet werden Lösungen von Alkalihydroxiden, Harnstoff, Guanidiniumsalzen oder geladenen Detergenzien, wie z.B. Alkyltrimethylammoniumsalzen oder Alkylsulfaten, verwendet. Insbesondere werden Lösungen von ≥ 0,2 M Natriumhydroxid, ≥ 2 M Harnstoff, ≥ 1M Guanidiniumhydrochlorid, ≥ 1M Guanidiniumthiocyanat oder ≥ 0,1%Natriumdodecylsulfat oder ≥ 0,1% Cetyltrimethylammoniumbromid verwendet. Für die Reinigung werden die stabilen Assoziate in den entsprechenden Lösungen resuspendiert und anschließend durch ein geeignetes Trennverfahren (z.B. Sedimentierung oder Filtration) von der Lösung getrennt. Anschließend werden die repetitiven Vorläuferproteine mit Wasser gewaschen und mit dem Fachmann geläufigen Methoden getrocknet.

Um die Peptide aus den repetitiven Vorläuferproteinen zu gewinnen, müssen diese Sequenzen aus den Vorläuferproteinen herausgespalten und von den Hilfssequenzen getrennt werden. Die Spaltung erfolgt an den in den repetitiven Vorläuferproteinen enthaltenen Spaltsequenzen. Methoden zur spezifischen Spaltung von Aminosäureketten sind in der Literatur beschrieben. Repetitive Vorläuferproteine können enzymatisch oder chemisch gespalten werden. Beispiele für Enzyme mit denen Aminosäureketten spezifisch gespalten werden können, sind Arg-C proteinase, Asp-N-Endopeptidase, Caspasen, Chymotrypsin, Clostripain, Enterokinase, Factor Xa, Glutamylendopeptidase, Granzyme B, LysC Lysylendopeptidase (Achromobacter proteinase I) LysN Peptidyl-Lys-Metalloendopeptidase, Pepsin, Prolin-Endopeptidase, Proteinase K, Staphylococcal peptidase I, Thermolysin, Thrombin, Trypsin. Beispiele für Chemikalien, mit denen Aminosäureketten spezifisch gespalten werden können sind BNPS-Skatole (2-(2'-Nitrophenylsulfenyl)-3-methyl-3-bromoinolenine), Bromcyan, Säuren, Hydroxylamin, Iodosobenzoesäure, NTCB (2-nitro-5-thiocyanobenzoesäure).

Insbesondere werden repetitive Vorläuferproteine chemisch gespalten, wie z.B. durch die Spaltung mit Hydroxylamin oder Säure. Für die Säurespaltung ist jede anorganische oder organische Säure geeignet mit einem pKₛ-Wert kleiner als 5 und größer als 0, bevorzugt kleiner als 4 und größer als 1. Insbesondere wird für die Spaltung 1 - 5 % Phosphorsäure oder 1 - 5 % Ameisensäure verwendet. Abhängig von den Säurespaltbedingungen findet eine einfache Spaltung zwischen den Aminosäuren Asp und Pro oder Asp und Xxx statt, wobei Xxx eine beliebige proteinogene Aminosäure darstellt oder es findet zunächst eine Spaltung zwischen den Aminosäuren Asp und Pro bzw. Asp und Xxx statt und anschließend eine vollständige Abspaltung des Aspartats von der in der Aminosäuresequenz des Peptids N-terminal vor dem Aspartat liegenden Aminosäure.

Die Spaltung kann mit dem gereinigten repetitiven Vorläuferprotein erfolgen oder mit einer Zellfraktion, die das repetitive Vorläuferprotein enthält (z.B. lösliche Bestandteile der Wirtszelle oder unlösliche Bestandteile einer Wirtszelle) oder mit intakten Wirtszellen, die das repetitive Vorläuferprotein enthalten. Nach der Spaltung muss die spaltende Substanz inaktiviert werden. Methoden dazu sind dem Fachmann bekannt.

Nach der Inaktivierung enthält der Spaltansatz u.a. das gewünschte Peptid, abgespaltene Hilfssequenzen sowie inaktivierte Spaltsubstanzen. Die Peptide können in dieser Lösung bereits ihre gewünschte Aktivität besitzen. Ist eine größere Reinheit erforderlich, so können nach der Spaltung die aus den repetitiven Vorläuferproteinen freigesetzten Peptide von den Hilfssequenzen abgetrennt werden. Ein Vorteil der selbstassemblierenden Hilfssequenzen ist, dass die Hilfssequenzen während oder nach der Spaltung assemblieren. Diese Assemblierung kann spontan während der Spaltung unter den gewählten Spaltungsbedingungen erfolgen, oder durch Zugabe von Stoffen, die die Assemblierung der Hilfssequenzen unterstützen. Solche assemblierungsfördernde Stoffe sind zum Beispiel kosmotrope Salze, die mindestens eine Ionenart enthalten, die entsprechend der Hofmeister-Reihe stärker kosmotrope Eigenschaften aufweist als Natrium- oder Chloridionen. Weitere assemblierungsfördernde Stoffe sind Säuren oder Laugen oder organische Lösungsmittel, die mit Wasser mischbar sind wie z.B. Alkohole. Die assemblierten Hilfssequenzen können durch Sedimentierung oder Filtration von dem löslichen freigesetzten Peptid abgetrennt werden. Weitere Reinigungsschritte können erforderlich sein, um restliche Protein- oder Peptidverunreinigungen oder Salze oder sonstige während oder nach der Spaltung zugesetzte Stoffe vom gewünschten Peptid abzutrennen. Dazu können beispielsweise chromatographische Methoden, Fällungen, Dialyse, 2-Phasen-Extraktionen und weitere dem Fachmann geläufige Verfahren eingesetzt werden.

Anschließend kann die peptidhaltige Lösung direkt für die gewünschte Anwendung eingesetzt werden, oder die Lösung kann mit dem Fachmann geläufigen Verfahren getrocknet werden (z.B. Sprühtrocknung oder Gefriertrocknung) und das entsprechende trockene Produkt verwendet werden.

Nach der Trocknung besteht die Möglichkeit, Verunreinigungen, die nicht vom Peptid abgetrennt werden können, solange dies in Wasser gelöst ist, durch Waschen mit Lösungsmitteln, in denen das Peptid unlöslich ist, zu entfernen. Geeignet dafür sind organische Lösungsmittel wie z.B. n-Hexan N-Methylpyrrolidon oder Gemische aus Lösungsmitteln und Säuren wie z.B. Mischungen aus n-Hexan und Essigsäure oder organische Säuren wie z.B. Essigsäure oder Hexansäure. Für diesen Reinigungsschritt wird das getrocknete Peptid in dem entsprechenden Lösungsmittel / -gemisch resuspendiert und anschließend durch Sedimentation oder Filtration wieder abgetrennt. Reste des Lösungsmittels / Lösungsmittelgemisches können durch Trocknung entfernt werden.

Die gewünschten Peptide können in der durch die Spaltung erhaltenen Form die gewünschte Aktivität besitzen. Es kann jedoch auch notwendig sein, die Peptide nach der Spaltung weiter zu modifizieren. Beispielsweise kann das Peptid amidiert, verestert, oxidiert, alkyliert oder mit beliebigen Molekülen chemisch verknüpft werden. Beispiele für Moleküle, die für solche Modifikationen verwendet werden können sind Alkohole, Alkoholcysteinester, Carbonsäuren, Thioester oder Maleinimide. Insbesondere werden für solche Modifikationen Moleküle verwendet, die die Hydrophobizität des Peptids erhöhen. Solche Moleküle können modifizierte oder nichtmodifizierte Alkylreste, wie oben definiert, enthalten. Bevorzugt enthalten solche Moleküle C₂-C₁₆-, insbesondere C₆-C₁₄-Alkylreste.Entsprechende Verfahren sind dem Fachmann bekannt. Die Modifizierung kann zu einem beliebigen Zeitpunkt erfolgen: z.B. direkt nach dem Zellaufschluss, nach Aufreinigung des Vorläuferproteins, nach der Spaltung des Vorläuferproteins oder nach der Aufreinigung des Peptids.

Peptidlösungen, die den gewünschten Reinheitsgrad aufweisen, können direkt verwendet werden. Alternativ können unterschiedliche Konservierungsmethoden für eine längerfristige Lagerung angewendet werden. Beispiele für Konservierungsmethoden sind Kühlung, Einfrieren, Zugabe von Konservierungsmitteln. Alternativ können die Peptide getrocknet werden. Beispiele für Trocknungsmethoden sind Lyophilisierung oder Sprühtrocknung. Getrocknete Peptide können anschließend gelagert werden. Für die Verwendung der Peptide wird die getrocknete Substanz in einem geeigneten Lösungsmittel, bevorzugt einer wässrigen Lösung gelöst. Diese wässrige Lösung kann Salze oder Puffersubstanzen oder keine weiteren Zusätze enthalten.

### 8. Definition verschiedener weiterer allgemeiner Begriffe

Unter einer von einer konkret offenbarten Sequenz "abgeleiteten" oder dazu "homologen" Sequenz, z.B. einer abgeleiteten Aminosäure- oder Nukleinsäuresequenz, wird erfindungsgemäß, wenn keine anderen Angaben gemacht werden, eine Sequenz verstanden, die mit der Ausgangssequenz eine Identität von mindestens 80% oder mindestens 90%, insbesondere 91%, 92%, 93%, 94%, 95%, 96%, 97%, 98% und 99% aufweist.

### EXPERIMENTELLER TEIL

Die universelle Anwendbarkeit der in der vorliegenden Erfindung beschriebene Methode für die Herstellung beliebiger Peptidsequenzen (Pep), wird anhand der Herstellung von drei in Sequenz und Aminosäurezusammensetzung unterschiedlicher Peptide (ZnO, P18, Min) gezeigt.

Werden keine anderen Angaben gemacht, so finden Standardmethoden der organischen und biochemischen Analytik sowie der rekombinaten Herstellung von Proteinen und Kultivierung von Mikroorganismen Anwendung.

### Beispiel 1: Herstellung des Peptids ZnO (SEQ ID NO:20)

Das Peptid ZnO ist ein von einer publizierten Sequenz abgeleitetes Peptid, dass die Bildung von Zinkoxidpartikeln beeinflusst (Umetsu et al. Adv. Mat. 17: 2571-75 (2005)). Ein synthetisches Gen ZnO₄ (SEQ ID NO:21) wurde unter Verwendung der Restriktionsendonukleasen BamHI und HindIII in den in Hummerich et al. Biochemistry 43; 13604-13612 (2004) beschriebenen Vektor pAZL kloniert und nach dem dort beschriebenen Protokoll dimerisiert und in den Vektor pET21 (Novagen) kloniert. Die anschließend in diesem Vektor vorliegende Sequenz kodiert für das repetitive Vorläuferprotein ZnO₈ (SEQ ID NO:22). Das repeptitive Vorläuferprotein enthält 8 Wiederholungseinheiten, die jeweils eine Kopie des Peptids ZnO und eine Hilfssequenz enthalten. Die Hilfssequenz enthält eine poly-Alanin Sequenz und verleiht dem repetitiven Vorläuferprotein selbstassemblierenden Eigenschaften. Zwischen den Hilfssequenzen und den Peptidsequenzen befinden sich die Aminosäuren Asp-Pro, die eine selektive Herausspaltung des ZnO-Peptids aus dem Vorläuferprotein mit Säure ermöglichen sollen. Die Expression erfolgte in dem Stamm *E.coli* BL21 [DE3] (Novagen).

Die Anzucht und Proteinsynthese erfolgte bei pO₂>20% und pH=6,8 im Fed-Batch Verfahren.

**Medium:**

| | |
|---|---|
| 8 Liter | Wasser |
| 25 g | Citronensäuremonohydrat |
| 40 g | Glycerin (99 %) |
| 125 g | Kaliumdihydrogenphosphat (KH₂PO₄) |
| 62,5 g | Ammoniumsulfat ((NH₄)₂SO₄) |
| 18,8 g | Magnesiumsulfatheptahydrat (MgSO₄ * 7H₂O) |
| 1,3 g | Calciumchloriddihydrat (CaCl₂ * 2H₂O) |
| 155 ml | Spurensalzlösung |
| | mit Wasser auf 9,8 Liter auffüllen |
| | pH-Wert mit 25%iger NaOH auf 6,3 einstellen |
| 3 ml | Tego KS 911 (Antifoam; Goldschmidt Produkte) |
| 1 g | Ampicillin |
| 190 mg | Thiaminhydrochlorid |
| 20 mg | Vitamin B12 |

**Spurensalzlösung:**

| | |
|---|---|
| 5 Liter | Wasser |
| 200,00 g | Citronensäuremonohydrat |
| 55,00 g | ZnSO₄ * 7H₂O |
| 42,50 g | (NH₄)₂Fe(SO₄)₂ * 6H₂O |
| 15,00 g | MnSO₄ * H₂O |
| 4,00 g | CuSO₄ * 5H₂O |
| 1,25 g | CoSO₄ * 7H₂O |

**Feeding-Lösung:**

| | |
|---|---|
| 1125 g | Wasser |
| 41,3 g | Citronensäure Monohydrat |
| 81,6 g | Natriumsulfat (Na₂SO₄) |
| 6,3 g | (NH₄)₂Fe(SO₄)₂ * 6H₂O |
| 4734 g | Glycerin 99,5 % |

Nach Aufbrauchen des im Grundmedium enthaltenen Glycerins wurde ein konstanter Feed bei einer Rate von 100 ml/h gestartet.

Die Proteinsynthese wurde durch Zugabe von 1 mM Isopropyl-β-D-thiogalactopyranosid induziert, nachdem die Bakterienkultur eine optische Dichte OD₆₀₀=60 erreichte. Zu diesem Zeitpunkt wurde die Temperatur der Kultur von 37°C auf 30°C gesenkt. 5 h nach Induktion wurden die Zellen geerntet.

Die Aufreinigung des ZnO₈ wurde nach folgendem Protokoll durchgeführt:
- Resuspension des Zellpellets in 5 ml pro Gramm Feuchtmasse 20 mM MOPS (3-(N-Morpholino)propansulfonsäure) pH 7,0
- Aufschluss der Zellen im Hochdruckhomogenisator bei 1400 bar
- Zentrifugation 30 min, 5000xg
- Inkubation des Überstandes 30 min bei 80°C
- Zentrifugation 30 min, 5000xg
- Fällung des ZnO₈ aus dem Überstand durch Zugabe von 1,8 M Ammoniumsulfat (Endkonzentration) über Nacht bei 4°C
- Waschen des Pellets mit 8 M Harnstoff
- 2 x Waschen des Pellets mit Wasser
- Lyophilisierung
- Das lyophilisierte ZnO₈ wurde bei -20°C gelagert.

Es wurde 2,2 g reines ZnO₈ pro Liter Kulturmedium gewonnen.

Für die Spaltung wurden 250 mg des lyophilisierten Vorläuferproteins ZnO₈ in 5mL 1% Ameisensäure resuspendiert und für 6 h bei 90°C inkubiert. Während dieser Inkubation löste sich das Lyophilisat und es entstand eine gelartige Substanz. Nach Abkühlen auf Raumtemperatur wurde die gelartige Substanz durch Sedimentation bei 18000 x g von den löslichen Bestandteilen abgetrennt. Die verbleibende Lösung wurde mit 2M NaOH neutralisiert. Anschließend wurde die Lösung lyophilisiert. Das Lyophilisat enthielt das gewünschte Spaltprodukt sowie aus der Neutralisation der Ameisensäure erhaltenes Natriumformiat.

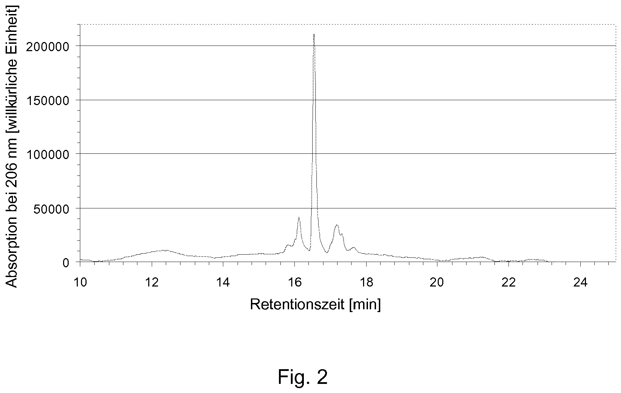

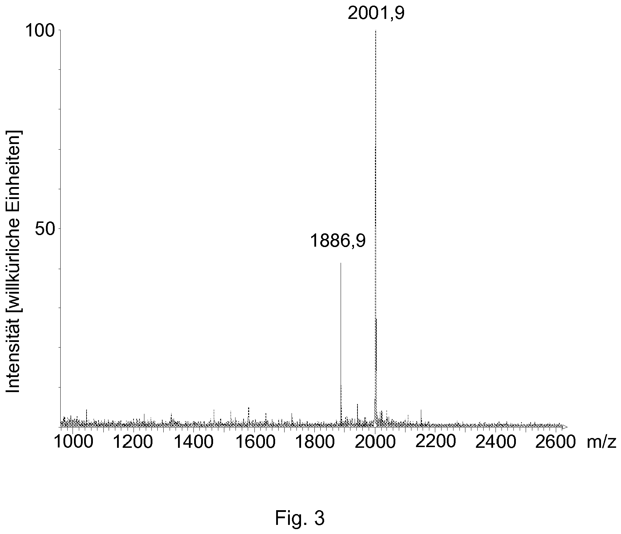

Das Lyophilisierte Produkt wurde mittels HPLC analysiert: Dazu wurde das Produkt mit einer Konzentration von 1 mg/ml in Wasser gelöst und mit einer Reversed Phase Chromatographiesäule (Jupiter Proteo 4,6 x 250 mm; Phenomenex) analysiert. Als Laufmittel wurde 0,1% Trifluoressigsäure in Wasser verwendet, das mit einem linearen Gradienten durch 0,1% Trifluoressigsäure in Acetonitril ersetzt wurde. Die Detektion erfolgte bei 206 nm (Figur 2). Für eine weitere Analyse wurden die Fraktionen des Hauptpeaks gesammelt und die darin enthaltene Substanz weiter untersucht. N-terminale Sequenzierung bestätigte, dass es sich bei dieser Komponente um das Peptid ZnO handelt. Untersuchungen mittels Massenspektrometrie (MALDI-TOF) ergaben eine Masse von 2002, die identisch mit der theoretischen Masse des ZnO-Peptids ist (Figur 3). Die HPLC Analyse ergab eine Reinheit von 62 % bezogen auf UV-aktive Komponenten.

### Beispiel 2: Herstellung des Peptids P18 (SEQ ID NO: 23)

Das Peptid P18 ist ein Peptid, das sich von einer hochwirksamen antimikrobiellen Peptidsequenz ableitet, die von Shin et al. J. Peptide Res. 58:504-14 (2001) beschrieben wurde. Ein synthetisches Gen AHeAP18₂ (SEQ ID NO:24) wurde unter Verwendung der Restriktionsendonukleasen BamHI und HindIII in den in Hummerich et al. Biochemistry 43; 13604-13612 (2004) beschriebenen Vektor pAZL kloniert und nach dem dort beschriebenen Protokoll dimerisiert und in den Vektor pET21 (Novagen) kloniert. Die anschließend in diesem Vektor vorliegende Sequenz kodiert für das repetitive Vorläuferprotein AHeAP18₄ (SEQ ID NO:25). Das repetitive Vorläuferprotein enthält 4 Wiederholungseinheiten, die jeweils eine Kopie des Peptids P18 und eine Hilfssequenz enthalten. Die Hilfssequenz enthält zwei poly-Alanin Sequenzen und verleiht dem repetitiven Vorläuferprotein selbstassemblierenden Eigenschaften. Außerdem enthält die Hilfssequenz eine negativ geladene helikale Schutzsequenz. Zwischen den Hilfssequenzen und den P18-Peptidsequenzen befinden sich die Aminosäuren Asp-Pro, die eine selektive Herausspaltung des P18-Peptids aus dem Vorläuferprotein mit Säure ermöglichen sollen. Die Expression erfolgte in dem Stamm *E.coli* BL21 [DE3] (Novagen).

Die Anzucht und Proteinsynthese erfolgte bei pO₂>20% und pH=6,8 im Fed-Batch Verfahren. Medium, Spurensalzlösung und Feeding-Lösung hatten die in Beispiel 1 beschriebene Zusammensetzung.

Nach Aufbrauchen des im Grundmedium enthaltenen Glycerins wurde ein konstanter Feed bei einer Rate von 100 ml/h gestartet.

Die Proteinsynthese wurde durch Zugabe von 1 mM Isopropyl-β-D-thiogalactopyranosid induziert, nachdem die Bakterienkultur eine optische Dichte OD₆₀₀=60 erreichte. 10 h nach Induktion wurden die Zellen durch Sedimentation bei 5000 x g für 30 min geerntet. Die Biofeuchtmasse betrug 1932g.

Die Biofeuchtmasse wurden nach folgendem Protokoll aufgereinigt:
- Resuspension des Zellpellets: pro g Biomasse wurden 6 g 20 mM Natriumphosphat-Puffer (pH 7,5) zugeben und durchmischt.
- Aufschluss der Zellen im Hochdruckhomogenisator bei 1500 bar
- Zugabe von Phosphorsäure bis pH-Wert = 3 ± 0,5
- 10 min Inkubation bei 23 °C unter Rühren
- Zentrifugation: 20 min, min. 5000 xg
- Resuspension des Pellets: pro g Feuchtmasse 25 mL 0,2 M NaOH zugeben, homogenisieren und 4 Stunden bei 23 °C unter Rühren inkubieren
- Neutralisation: pH-Wert von 8,5 ± 0,5 mit 85%iger H₃PO₄ einstellen
- Zentrifugation: 20 min, min. 5000 xg
- Das Pellet bestehend aus gewaschenen Inclusion bodies, die das P18-Vorläuferprotein enthalten, wurde mittels 2 % iger H₃PO₄ hydrolysiert bzw. gespalten.
   Spaltungsbedingungen:
   i. Pro g Pellet 5 mL H₃PO₄ einsetzen
   ii. Homogenisieren
   iii. 16 Stunden bei 90 °C unter Schütteln inkubieren.
- Spaltungsansatz abkühlen lassen.
- Zentrifugation: 20 min, min. 5000 xg
- Neutralisation: pH-Wert von 5,5 ± 0,5 mit 10 M NaOH einstellen
- Zentrifugation: 20 min, min. 5000 xg
- Überstand mit Wasser verdünnen bis Leitfähigkeit kleiner 10 mS/cm
- P18 aus Überstand über Kationenaustauschchromatographie (Fractogel COO; Merck) aufreinigen; Elution mit 450 mM NaCl
- Peptidhaltige Fraktionen poolen und mit Wasser verdünnen, bis Leitfähigkeit kleiner 10 mS/cm
- P18 aus gepoolten Fraktionen über Kationenaustauschchromatographie (Fractogel COO; Merck) aufreinigen; Elution mit 50 mM HCl
- Das Eluat wurde mit 2 M NaOH neutralisiert.
- Die neutralisierte Lösung wurde lyophilisiert.

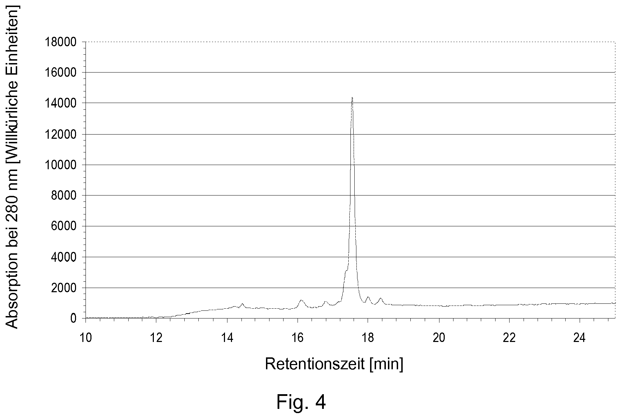

Das lyophilisierte Produkt wurde mittels HPLC analysiert: Dazu wurde das Produkt mit einer Konzentration von 1 mg/ml in Wasser gelöst und mit einer Reversed Phase Chromatographiesäule (Jupiter Proteo 4,6 x 250 mm; Phenomenex) analysiert. Als Laufmittel wurde 0,1% Trifluoressigsäure in Wasser verwendet, das mit einem linearen Gradienten durch 0,1% Trifluoressigsäure in Acetonitril ersetzt wurde. Die Detektion erfolgte bei 280 nm (Figur 4). Für eine weitere Analyse wurden die Fraktionen des Hauptpeaks gesammelt und die darin enthaltene Substanz weiter untersucht. N-terminale Sequenzierung bestätigte, dass es sich bei dieser Komponente um das Peptid P18 handelt.

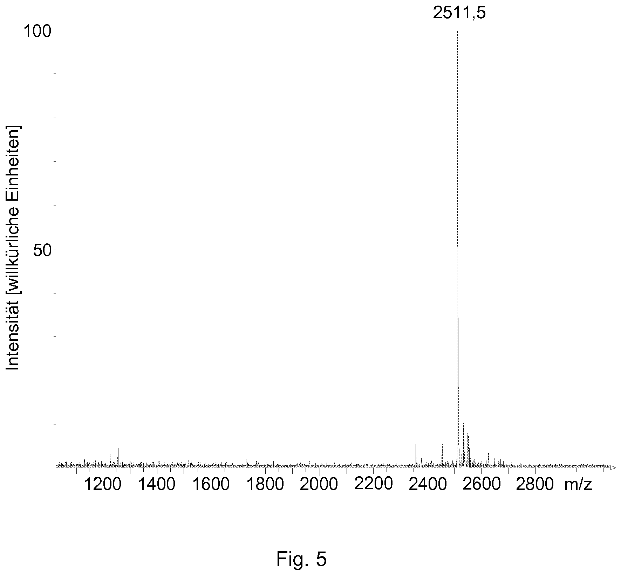

Untersuchungen mittels Massenspektrometrie (MALDI-TOF) ergaben für das Peptid eine Masse von 2512, die identisch mit der theoretischen Masse des P18-Peptids ist (Figur 5). Die HPLC Analyse ergab eine Reinheit von 85 % bezogen auf UV-aktive Komponenten. Aus 30 g Biofeuchtmasse wurden 52 mg P18-Peptid erhalten. Es können somit pro Liter Fermentationskultur ca. 330 mg reines P18-Peptid gewonnen werden.

Um die Aktivität des P18-Peptids zu untersuchen, wurden E. coli B Kulturen in LB Medium (5 g/l Hefeextrakt; 10 g/l Trypton 5 g/l Natriumchlorid), die eine optische Dichte von 0,1 gemessen bei 600 nm aufwiesen, mit unterschiedlichen Konzentrationen des P18 Peptids unter Schütteln bei 37°C inkubiert. Das Bakterienwachstum wurde durch Messung der optischen Dichte nach 24 h verfolgt. Eine vollständige Hemmung des Wachstums (optische Dichte bei 600 nm nach 24 h < 0,15) wurde bei einer Peptidkonzentration ab 31 ppm erreicht. Eine weitere Verbesserung der antimikrobiellen Aktivität konnte durch Amidierung der C-terminalen Carboxylgruppe erreicht werden. Dazu wurden die Carboxylgruppen mit 1-Ethyl-3-(3-dimethylaminopropyl)carbodiimid-Hydrochlorid und N-Hydroxy-Sulfosuccinimid aktiviert und durch anschließende Zugabe von Ammoniak amidiert.

### Beispiel 3: Herstellung des Peptids Min (SEQ ID NO: 26)

Ein synthetisches Gen AEMin₄ (SEQ ID NO:27) wurde unter Verwendung der Restriktionsendonukleasen BamHI und HindIII in den in Hummerich et al. Biochemistry 43; 13604-13612 (2004) beschriebenen Vektor pAZL kloniert und nach dem dort beschriebenen Protokoll dimerisiert und in den Vektor pET21 (Novagen) kloniert. Die anschließend in diesem Vektor vorliegende Sequenz kodiert für das repetitive Vorläuferprotein AEMin₈ (SEQ ID NO:28). Das repetitive Vorläuferprotein enthält 8 Wiederholungseinheiten, die jeweils eine Kopie des Peptids Min und eine Hilfssequenz enthalten. Die Hilfssequenz enthält eine poly-Alanin Sequenz und verleiht dem repetitiven Vorläuferprotein selbstassemblierenden Eigenschaften. Außerdem enthält die Hilfssequenz eine negativ geladene Schutzsequenz. Zwischen den Hilfssequenzen und den Peptidsequenzen befinden sich die Aminosäuren Asp-Pro, die eine selektive Herausspaltung des P18-Peptids aus dem Vorläuferprotein mit Säure ermöglichen sollen. Die Expression erfolgte in dem Stamm *E. coli* BL21 [DE3] (Novagen).

Die Anzucht und Proteinsynthese erfolgte bei pO₂>20% und pH=6,8 im Fed-Batch Verfahren. Medium, Spurensalzlösung und Feeding-Lösung hatten die in Beispiel 1 beschriebene Zusammensetzung.

Nach Aufbrauchen des im Grundmedium enthaltenen Glycerins wurde ein konstanter Feed bei einer Rate von 100 ml/h gestartet.

Die Proteinsynthese wurde durch Zugabe von 1 mM Isopropyl-β-D-thiogalactopyranosid induziert, nachdem die Bakterienkultur eine optische Dichte OD₆₀₀=60 erreichte. Zu diesem Zeitpunkt wurde die Temperatur der Kultur von 37°C auf 30°C gesenkt. 5 h nach Induktion wurden die Zellen geerntet.

Die Aufreinigung des AEMin₈ wurde nach folgendem Protokoll durchgeführt:
- Resuspension des Zellpellets in 5 ml pro Gramm Feuchtmasse 20 mM MOPS (3-(N-Morpholino)propansulfonsäure) pH 7,0
- Aufschluss der Zellen im Hochdruckhomogenisator bei 1400 bar
- Zentrifugation 30 min, 5000xg
- Inkubation des Überstandes 20 min bei 80°C
- Zentrifugation 30 min, 5000xg
- Fällung des AEMin₈ aus dem Überstand durch Zugabe von 2 M Ammoniumsulfat (Endkonzentration) über Nacht bei 4°C
- Waschen des Pellets mit 8 M Harnstoff
- 2 x Waschen des Pellets mit Wasser
- Lyophilisierung
- Das lyophilisierte AEMin₈ wurde bei -20°C gelagert.

Es wurde 0,4 g reines AEMin₈ pro Liter Kulturmedium gewonnen.

Für die Spaltung wurden 250 mg des lyophilisierten Vorläuferproteins AEMin₈ in 12,5mL 1% Phosphorsäure resuspendiert und für 8 h bei 90°C inkubiert. Nach Abkühlen auf Raumtemperatur wurden unlösliche Substanzen durch Sedimentation bei 18000 x g von den löslichen Bestandteilen abgetrennt. Die verbleibende Lösung wurde mit 2M NaOH neutralisiert. Anschließend wurde die Lösung lyophilisiert. Das Lyophilisat enthielt das gewünschte Spaltprodukt sowie aus der Neutralisation der Phosphorsäure erhaltenes Natriumhydrogenphosphat.

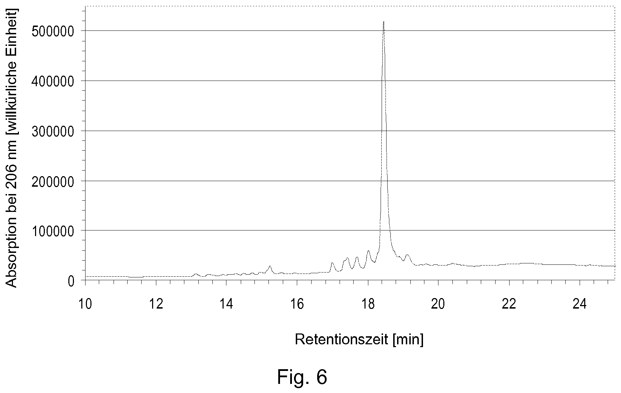

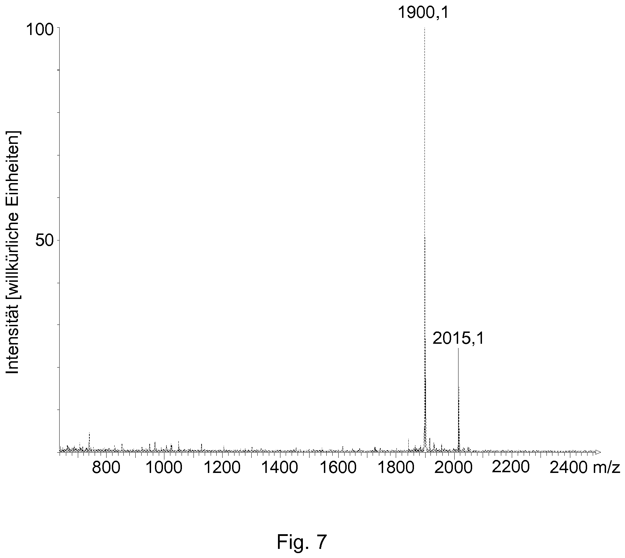

Das lyophilisierte Produkt wurde mittels HPLC analysiert: dazu wurde das Produkt mit einer Konzentration von 1 mg/ml in Wasser gelöst und mit einer Reversed Phase Chromatographiesäule (Jupiter Proteo 4,6 x 250 mm; Phenomenex) analysiert. Als Laufmittel wurde 0,1% Trifluoressigsäure in Wasser verwendet, das mit einem linearen Gradienten durch 0,1% Trifluoressigsäure in Acetonitril ersetzt wurde. Die Detektion erfolgte bei 206 nm (Figur 6). Für eine weitere Analyse wurden die Fraktionen des Hauptpeaks gesammelt und die darin enthaltene Substanz weiter untersucht. N-terminale Sequenzierung bestätigte, dass es sich bei dieser Komponente um das Peptid Min handelt. Untersuchungen mittels Massenspektrometrie (MALDI-TOF) ergaben für das Peptid eine Masse von 1900, die identisch mit der theoretischen Masse des Min-Peptids ist (Figur7). Die HPLC Analyse ergab eine Reinheit von 68 % bezogen auf UV-aktive Komponenten.

### Beispiel 4: Optimierung der Herstellung des Peptids P18 (SEQ ID NO: 23)

Um die Ausbeute des Peptids P18 aus Beispiel 2 zu erhöhen wurde der Einfluss unterschiedlicher Aux-Sequenzen auf die Peptidausbeute untersucht. Eine deutliche Steigerung der Expression und Gesamtausbeute wurde mit dem synthetischen Gen A-He2AP18₂ (SEQ ID NO:74) erreicht, das nach Klonierung in den Vektor pET21 entsprechend dem Beispiel 2 für das Vorläuferprotein mit der SEQ ID NO:75 codiert. Die Fermentation erfolgte unter den in Beispiel 2 beschriebenen Bedingungen.

Die Biofeuchtmasse wurden nach folgendem Protokoll aufgereinigt:
- Resuspension des Zellpellets: pro g Biomasse wurden 6 g Wasser zugeben und durchmischt
- Aufschluss der Zellen im Hochdruckhomogenisator bei 1500 bar
- Zugabe von Phosphorsäure bis pH-Wert = 3 ± 0,5
- 10 min Inkubation bei 23 °C unter Rühren
- Zentrifugation: 20 min, min. 5000 xg
- Resuspension des Pellets: pro g Feuchtmasse 20 mL 0,4 M NaOH zugeben, homogenisieren und 1 Stunde bei 23 °C unter Rühren inkubieren
- Neutralisation: pH-Wert von 8,5 ± 0,5 mit 1 M Kaliumphosphatpuffer pH 6.0 einstellen
- Zentrifugation: 20 min, min. 5000 xg
- Pellet bestehend aus gewaschenen Inclusion bodies, die das P18-Vorläuferprotein enthalten, wurde mittels 2 % iger H₃PO₄ hydrolysiert bzw. gespalten
   Spaltungsbedingungen:
   i. pro g Pellet 7 mL H₃PO₄ einsetzen
   ii. Homogenisieren
   iii. 16 Stunden bei 90 °C unter Schütteln inkubieren
- Spaltungsansatz abkühlen lassen
- Zentrifugation: 20 min, min. 5000 xg
- pH-Wert von 4,0 ± 0,5 mit 25% NaOH einstellen
- Zentrifugation: 20 min, min. 5000 xg
- Überstand mit Wasser verdünnen bis Leitfähigkeit kleiner 30 mS/cm
- P18 aus Überstand über Kationenaustauschchromatographie (SP-Sepharose High Performance; GE Healthcare) aufreinigen; Waschpuffer: 10 mM Natriumacetat-Puffer pH 4 + 450 mM NaCl; Elutionspuffer: 10 mM Natriumacetat-Puffer pH 4 + 1100 mM NaCl
- Fällung des Peptids durch Zugabe von 25% NaOH zum Eluat bis pH= 10,5 ± 0,3
- Zentrifugation: 20 min, min. 5000 xg
- Resuspension des Pellets in 5 ml Wasser pro Gramm Feuchtmasse
- Lösen des Peptids: Zugabe von Essigsäure bis pH 6,0 pH= 10,5 ± 0,5
- Lyophilisierung

Auf die beschriebene Art können pro Liter Fermentationskultur ca. 1g reines P18-Peptid gewonnen werden.

### Beispiel 5: Herstellung des Peptids SEQ ID NO: 6

Die in Beispiel 1 bis 4 aufgeführten Peptide wurden durch Säurespaltung aus dem repetitiven Vorläuferprotein erhalten. Dabei wird die Peptidbindung zwischen einem Aspartat und einem Prolin hydrolysiert. Entsprechend beginnt, wie in Beispiel 1 gezeigt, die Peptisequenz N-terminal mit einem Prolin und endet C-terminal mit einem Aspartat. Unter bestimmten Umständen kann das C-terminale Aspartat, wie in Beispiel 2 und 3 gezeigt, ebenfalls abgespalten werden und erlaubt damit, Peptidsequenzen mit einer frei wählbaren C-terminalen Sequenz herzustellen.

Um zu zeigen, dass mit dem erfindungsgemäßen Verfahren auch Peptide hergestellt werden können, bei denen der N-terminus nicht mit einem Prolin beginnt sondern bei denen die erste N-terminale Aminosäure frei wählbar ist, wurde das Peptid mit der SEQ ID NO:6 hergestellt. Dazu wurde entsprechend Beispiel 4 unter Verwendung des synthetischen Gens AHe2AP18₂-P-G (SEQ ID NO:76) das Vorläuferprotein mit der SEQ ID NO:77 hergestellt. Dieses Vorläuferprotein unterscheidet sich von dem Vorläuferprotein SEQ ID NO:75 aus Beispiel 4 lediglich darin, dass die N-terminalen Aminosäuren des P18-Peptids Pro-Gly und das C-terminale Gly deletiert sind. Die Klonierung und Fermentation erfolgte unter den in Beispiel 4 beschriebenen Bedingungen.

Die Biofeuchtmasse wurden nach folgendem Protokoll aufgereinigt:
- Resuspension des Zellpellets: pro g Biomasse wurden 6 g Wasser zugeben und durchmischt
- Aufschluss der Zellen im Hochdruckhomogenisator bei 1500 bar
- Zentrifugation: 20 min, min. 5000 xg
- Pellet bestehend aus Inclusion bodies, die das P18-Vorläuferprotein enthalten, wurde mittels 5 % iger H₃PO₄ hydrolysiert bzw. gespalten
   Spaltungsbedingungen:
   i. Pro g Pellet 5 mL H₃PO₄ einsetzen
   ii. Homogenisieren
   iii. 16 Stunden bei 90 °C unter Schütteln inkubieren
- Spaltungsansatz abkühlen lassen
- Zentrifugation: 20 min, min. 5000 xg
- pH-Wert von 4,0 ± 0,5 mit 25% NaOH einstellen
- Zentrifugation: 20 min, min. 5000 xg
- Überstand mit Wasser verdünnen bis Leitfähigkeit kleiner 30 mS/cm
- Peptid aus Überstand über Kationenaustauschchromatographie (SP-Sepharose High Performance; GE Healthcare) aufreinigen; Waschpuffer: 10 mM Natriumacetat-Puffer pH 4 + 450 mM NaCl; Elutionspuffer: 10 mM Natriumacetat-Puffer pH 4 + 1100 mM NaCl
- Fällung des Peptids durch Zugabe von 25% NaOH zum Eluat bis pH= 10,5 ± 0,3
- Zentrifugation: 20 min, min. 5000 xg
- Resuspension des Pellets in 5 ml Wasser pro Gramm Feuchtmasse
- Lösen des Peptids durch Zugabe von Essigsäure bis pH = 6,0 ± 0,5
- Lyophilisierung

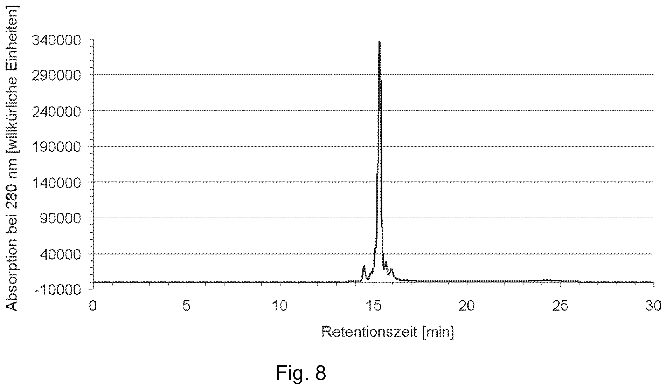

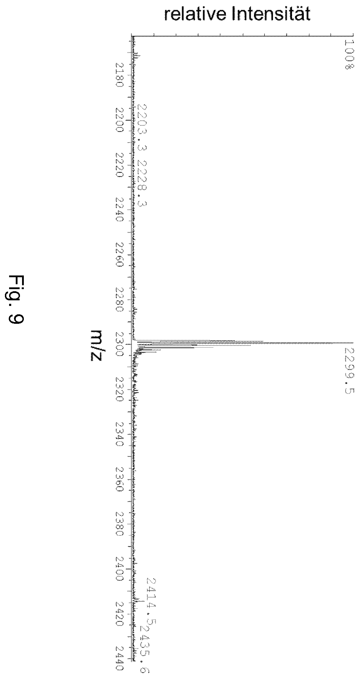

Das lyophilisierte Produkt wurde mittels HPLC analysiert Dazu wurde das Produkt mit einer Konzentration von 1 mg/ml in Wasser gelöst und mit einer Reversed Phase Chromatographiesäule (Jupiter Proteo 4,6 x 250 mm; Phenomenex) analysiert. Als Laufmittel wurde 0,1% Trifluoressigsäure in Wasser verwendet, das mit einem linearen Gradienten durch 0,1% Trifluoressigsäure in Acetonitril ersetzt wurde. Die Detektion erfolgte bei 280 nm (Figur 8). Für eine weitere Analyse wurden die Fraktionen des Hauptpeaks gesammelt und die darin enthaltene Substanz weiter untersucht. Untersuchungen mittels Massenspektrometrie (MALDI-TOF) ergaben für das Peptid eine Masse von 2300,6, die gleich der theoretischen Masse des Peptids SEQ ID NO: 6 ist (Figur 9).

### Beispiel 6: Amidierung von lyophilisiertem Peptid P18 (SEQ ID NO: 23)

In bestimmten Fällen kann es vorteilhaft für die Aktivität eines Peptids sein, wenn der C-Terminus nicht als freie Carboxylgruppe vorliegt sondern amidiert ist. Um dies zu zeigen wurde lyophilisiertes P18-Peptid aus Beispiel 4 nach folgendem Protokoll amidiert:
∘ 10 mg/ml P18 Peptid
∘ 30 % EtOH
∘ 10 mM 2-(N-morpholino)ethansulfonsäure pH5,0
∘ 3M Ammoniumchlorid
∘ 2,5 mM N-Hydroxysuccinimid
∘ 50 mM 1-Ethyl-3-(3-dimethylaminopropyl)carbodiimid
∘ Inkubation 2h @ RT
∘ Neutralisierung mit NaOH pH 7,0

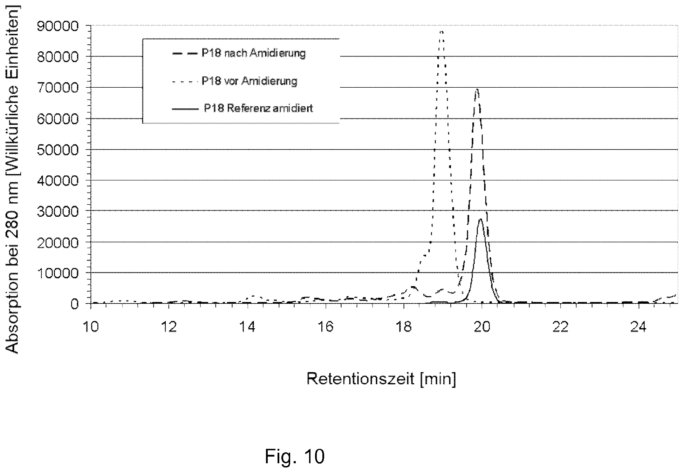

Die amidierte Probe wurde mittels HPLC mit einer Luna SCX 5µ 100A Chromatographiesäule (Phenomenex, Torrance, CA, USA) analysiert: Als Laufmittel wurde 20mM KH₂PO₄ pH2,5 mit 25% Acetonitril verwendet, das mit einem linearen Gradienten durch 20mM KH₂PO₄ pH2,5; 25% Acetonitril und 1 M KCl ersetzt wurde. Die Detektion erfolgte bei 280 nm (Figur 10). Zum Vergleich ist in Figur 10 das Chromatogramm eins chemisch synthetisierten und amidierten Referenz-Peptids mit der Sequenz des Peptids "P18" (in Auftrag hergestellt von der Bachem AG, Bubendorf, Schweiz) dargestellt.

Das Peptid wurde weiter über Kationenaustauschchromatographie wie in Beispiel 4 beschrieben aufgereinigt.

### Beispiel 7: Herstellung des Peptids P18 (SEQ ID NO: 23) mit integrierter Amidierung

Um die Kosteneffizienz der Herstellung von amidiertem Peptid P18 zu verbessern, wurde anstelle einer Amidierung im Anschluss einer Peptidaufreinigung, wie sie in Beispiel 6 beschrieben ist, die Amidierung in das Aufarbeitungsverfahren integriert. Dazu wurde das Vorläuferprotein SEQ ID NO:75 entsprechend Beispiel 4 durch Fermentation gewonnen und das Peptid durch Säurespaltung aus dem Vorläuferprotein freigesetzt.

Daraufhin wurden folgende Schritte durchgeführt:
- Spaltungsansatz abkühlen lassen.
- Zentrifugation: 20 min, min. 5000 xg
- pH-Wert von 10,5 ± 0,5 mit 25% NaOH einstellen
- Zentrifugation: 20 min, min. 5000 xg
- Pellet in 3 ml Ethanol pro g Feuchtmasse lösen
- Abzentrifugieren; Bestimmung von Peptid im Überstand (Verdünnen)
- Mischung folgender Komponenten:
   a. 12,5 ml gelöstes Peptid in Ethanol
   b. 4,2 ml Wasser
   c. Zugabe 400 µl 500mM 2-(N-morpholino)ethansulfonsäure
   d. einstellen auf pH 5,0 mit HCl
   e. Zugabe 2,14 g Ammoniumchlorid
   f. 200 µl 500 mM N-Hydroxysuccinimid
   g. 1 ml 1 M 1-Ethyl-3-(3-dimethylaminopropyl)carbodiimid
- Inkubation 2h bei RT
- Ansatz mit Wasser verdünnen bis Leitfähigkeit kleiner 30 mS/cm
- modifiziertes P18 Peptid über Kationenaustauschchromatographie (SP-Sepharose High Performance; GE Healthcare) aufreinigen; Waschpuffer: 10 mM Natriumacetat-Puffer pH 4 + 450 mM NaCl; Elutionspuffer: 10 mM Natriumacetat-Puffer pH 4 + 1100 mM NaCl
- Fällung des Peptids durch Zugabe von 25% NaOH zum Eluat bis pH= 10,5 ± 0,3
- Zentrifugation: 20 min, min. 5000 xg
- Resuspension des Pellets in 5 ml Wasser pro Gramm Feuchtmasse
- Lösen des Peptids: Zugabe von Essigsäure bis pH 6,0 ± 0,5
- Lyophilisierung

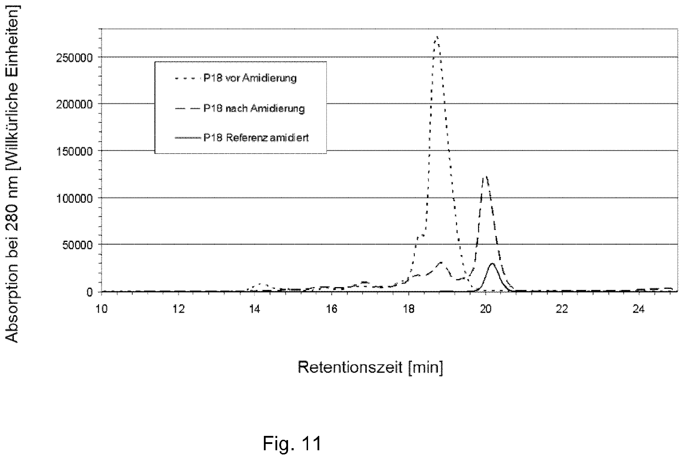

Die amidierte Probe wurde mittels HPLC mit einer Luna SCX 5µ 100A Chromatographiesäule (Phenomenex, Torrance, CA, USA) analysiert. Als Laufmittel wurde 20mM KH₂PO₄ pH2,5 mit 25% Acetonitril verwendet, das mit einem linearen Gradienten durch 20mM KH₂PO₄ pH2,5; 25% Acetonitril und 1 M KCl ersetzt wurde. Die Detektion erfolgte bei 280 nm (Figur 11). Zum Vergleich ist in Figur 11 das Chromatogramm eins chemisch synthetisierten und amidierten Referenz-Peptids mit der Sequenz des Peptids "P18" (in Auftrag hergestellt von der Bachem AG, Bubendorf, Schweiz) dargestellt.

**Übersicht über erfindungsgemäße Sequenzen**

| SEQ ID NO: | Sequenz | Bezeichnung Typ |
|---|---|---|
| 1 | GSSAAAAAAAASGP | SA |
| 2 | GSSAAAAAAAAASGP | SA |
| 3 | GSAAAAAAAASGP | SA |
| 4 | GSVVVVVVVVSGP | SA |
| 5 | GSVVAAVVAASGP | SA |
| 6 | KWKLFKKIPKFLHLAKKF | Pep |
| 7 | X₁ X₂K X₃ X₄ X₅KIP X₁₀ KFX₆X₇ X₈ AX₉KF | Pep |
| 8 | X₁ X₂K X₃ X₄ X₅KIP X₁₁ X₁₂ KFX₆X₇ X₈ AX₉KF | Pep |
| 9 | KWKLFKKIPPKFLHLAKKF | Pep |
| 10 | RWKLFKKIPKFLHLAKKF | Pep |
| 11 | FKKLFKKIPKFLHAAKKF | Pep |
| 12 | KWKLLKKIPKFKKLALKF | Pep |
| 13 | KWKLFKKIPKFLHAAKKF | Pep |
| 14 | KWKKFLKIPKFLHAAKKF | Pep |
| 15 | KWKKLLKIPKFLHAAKKF | Pep |
| 16 | FEEISEFLQSLEEF | SU helikal |
| 17 | EWELFEEISEFLQSLEEF | SU helikal |
| 18 | ELFEELAEFLQQLEEFIE | SU helikal |
| 19 | LFEELQEFLQALEELAQFALQFLAAFLQFS | SU helikal |
| 20 | PEAHVMHKVAPRPGGGSCGD | Pep (ZnO) |
| 21 | | ZnO₄ Konstrukt |
| | | |
| 22 | | ZnO₈ Vorläuferpeptid |
| 23 | PGKWKLFKKIPKFLHLAKKFG | Pep (P18) |
| 24 | | AheAP18₂ Konstrukt |
| 25 | | AheAP18₄ Vorläuferpeptid |
| 26 | PGERKRLIGCSVMTKPAG | Pep (Min) |
| 27 | | AEMin₄ Konstrukt |
| | | |
| 28 | | AEMin₈ Vorläuferpeptid |
| 29 | NPSSLFRYLPSD | Pep |
| 30 | HGGGHGHGGGHG | Pep |
| 31 | HYPTLPLGSSTY | Pep |
| 32 | ALSPHSAPLTLY | Pep |
| 33 | SAGRLSA | Pep |
| 34 | TLPNHTV | Pep |
| 35 | HTSKLGI | Pep |
| 36 | MSPHPHPRHHHTGGGK | Pep |
| 37 | EAHVMHKVAPRPGGGSC | Pep (ZnO kurz) |
| 38 | SSKKSGSYSGSKGSRRIL | Pep |
| 39 | PYAYMKSRDIESAQSDEEVELRDALAD | Pep |
| 40 | PGYGYYKNRNAEPAAAEAVD | Pep |
| 41 | PGKSRDIESAQSDEEVELRD | Pep |
| 42 | PGKSRDAEPAAAGEEVD | Pep |
| 43 | SSKKSGSYSGSKGSRRILGGGNPSSLFRYLPSD | Pep |
| 44 | MSPHPHPRHHHTGGGNPSSLFRYLPSD | Pep |
| 45 | NPSSLFRYLPSDGGGRREEWWDDRREEWWDD | Pep |
| 46 | MSPHPHPRHHHTGGGHGGGHGHGGGHG | Pep |
| 47 | SSKKSGSYSGSKGSRRILGGGHGGGHGHGGGHG | Pep |
| 48 | SSKKSGSYSGSKGSRRILGGGHYPTLPLGSSTY | Pep |
| 49 | SSKKSGSYSGSKGSRRILGGGSAGRLSA | Pep |
| 50 | RREEWWDDRREEWWDD | Pep |
| 51 | MKQLADSLMQLARQVSRLESA | Pep |
| 52 | MKQLADSLHQLARQVSRLEHA | Pep |
| 53 | LMQLARQMKQLADSLMQLARQVSRLESA | Pep |
| 54 | MKELADSLMQLARQVDRLESA | Pep |
| 55 | MKQLADSLHQLAHQVSHLEHA | Pep |
| 56 | PHFRFSFSP | Pep |
| 57 | PHFSFSFSP | Pep |
| 58 | PSFRFSFSP | Pep |
| 59 | MEELADSLEELARQVEELESA | Pep |
| 60 | MKKLADSLKKLARQVKKLESA | Pep |
| 61 | PHFHFSFSP | Pep |
| 62 | PHFSFHFSP | Pep |
| 63 | MKQLADSLHQLAHKVSHLEHA | Pep |
| 64 | EISALEKEISALEKEISALEK | Pep |
| 65 | KISALKEKISALKEKISALKE | Pep |
| 66 | RADARADARA DARADA | Pep |
| 67 | VKVKVKVKVG PPTKVKVKVK V | Pep |
| 68 | EAEPED | SU nicht helikal |
| 69 | PGKWKLFKKIPKFLHLAKKFGD | Pep |
| 70 | PGERKRLIGCSVMTKPAGD | Pep |
| 71 | PGKWKLFKKIPKFLHLAKKFGN | Pep |
| 72 | ERKRLIGCSVMTKPA | Pep (Min kurz) |
| 73 | GAAAAAAAASGP | SA |
| 74 | | AHe2AP18₂ Konstrukt |
| 75 | | AHe2AP18₄ Vorläuferpeptid |
| | | |
| 76 | | AHe2AP18-P-G₂ Konstrukt |
| 77 | | AHe2AP18-P-G₄ Vorläuferpeptid |

| | | |
|---|---|---|
| SA = selbstassemblierende Sequenz SU = Schutzpeptid Pep = herzustellendes Peptid | | |

In Ergänzung der oben beschriebenen konkreten Pep-Aminosäuresequenzen können die aufgelisteten Sequenzen C-terminal und/oder N-terminal durch Addition spezifischer Spaltsequenzen (z.B. für eine Säurespaltung zwischen den Resten "DP"; oder eine Hydroxylaminspaltung zwischen den Resten "NG") oder durch die aus derartigen Spaltungen resultierenden, verbleibenden Aminosäurereste abgewandelt sein. Gegebenenfalls kann auch zusätzlich zwischen Spaltsequenz und Pep-Sequenz ein Spacer-Rest, wie z.B. ein G-Rest, eingeschoben sei. Insbesondere sind folgende Abwandlungen obiger Pep-Sequenzen zu nennen, die aus der Säure- oder Hydroxylaminspaltung erfindungsgemäß hergestellter Pep-Sequenzen resultieren können:
N-terminal: Addition eines PG-, P- oder G-Rests;
C-terminal: Addition eines Rests -GD; -GN; -G; -N oder -D
Derartige Abwandlungen gelten insbesondere für jede einzelne der obigen Pep-Sequenzen, insbesondere gemäß SEQ ID NO:6 bis 15, 29 bis 67 und 72

Auf die Offenbarung der hierin genannten Literaturstellen wird ausdrücklich Bezug genommen

## Patentansprüche

1. Vorläuferprotein, umfassend eine spaltbare repetitive Abfolge von gewünschten Peptid (Pep)-Elementen und Hilfspeptid (Aux)-Elementen, der allgemeinen Formel
(Pep-Aux)ₓ oder
(Aux-Pep)ₓ
worin x >1 ist, wobei
die Aux-Elemente gleich oder verschieden sind und Aminosäuresequenzelemente umfassen, die dem Vorläuferprotein selbstassemblierende Eigenschaften verleihen, wobei das Aux-Element ein selbstassemblierendes Peptid ((SA)-Element) umfasst
wobei das SA-Element wenigstens eines der folgenden Sequenzmotive enthält:
Aₙ (Motiv 1)
(GA)ₘ (Motiv 2)
Vₙ (Motiv 3)
(VA)ₘ (Motiv 4)
(VVAA)ₒ (Motiv 5)
worin A für Alanin, G für Glycin, V für Valin, n für einen ganzzahligen Wert von 8 bis 12, m für einen ganzzahligen Wert von 4 bis 10, und o für einen ganzzahligen Wert von 2 bis 6 steht; und
die Pep-Elemente gleich oder verschieden sind und die Aminosäuresequenz gleicher oder verschiedener Peptidmoleküle mit einer Sequenzlänge von 5-70 Aminosäureresten umfassen, und die Pep-Elemente von Spaltsequenzen flankiert sind, die eine spezifische Herausspaltung der Pep-Elemente aus dem Vorläuferprotein ermöglichen, wobei
die Elemente Pep und Aux miteinander peptidisch verknüpft sind und die peptidische Verknüpfung chemisch spezifisch spaltbar ist, wobei die Spaltsequenzen ein Sequenzmotiv, ausgewählt unter NG und DP, umfassen.

2. Vorläuferprotein, umfassend eine spaltbare repetitive Abfolge von gewünschten Peptid (Pep)-Elementen und Hilfspeptid (Aux)-Elementen, der allgemeinen Formel
(Pep-Aux)ₓ oder
(Aux-Pep)ₓ
worin x >1 ist, wobei
die Aux-Elemente gleich oder verschieden sind und Aminosäuresequenzelemente umfassen, die dem Vorläuferprotein selbstassemblierende Eigenschaften verleihen, wobei das Aux-Element ein selbstassemblierendes Peptid ((SA)-Element) umfasst
wobei das SA-Element wenigstens eines der folgenden Sequenzmotive enthält:
Aₙ (Motiv 1)
(GA)ₘ (Motiv 2)
Vₙ (Motiv 3)
(VA)ₘ (Motiv 4)
(VVAA)ₒ (Motiv 5)
worin A für Alanin, G für Glycin, V für Valin, n für einen ganzzahligen Wert von 8 bis 12, m für einen ganzzahligen Wert von 4 bis 10, und o für einen ganzzahligen Wert von 2 bis 6 steht; und
die Pep-Elemente gleich oder verschieden sind und die Aminosäuresequenz gleicher oder verschiedener Peptidmoleküle mit einer Sequenzlänge von 5-70 Aminosäureresten umfassen, und die Pep-Elemente von Spaltsequenzen flankiert sind, die eine spezifische Herausspaltung der Pep-Elemente aus dem Vorläuferprotein ermöglichen, wobei
die Elemente Pep und Aux miteinander peptidisch verknüpft sind und die peptidische Verknüpfung chemisch oder enzymatisch spezifisch spaltbar ist, wobei
a) das Pep-Element eine Aminosäuresequenz, ausgewählt unter den kationischen Aminosäuresequenzen SEQ ID NO: 6 bis SEQ ID NO:15, SEQ ID NO: 23, SEQ ID NO: 26 und SEQ ID NO: 69 bis SEQ ID NO: 72 umfasst, oder
b) das Pep-Element eine Aminosäuresequenz, ausgewählt unter den Aminosäuresequenzen SEQ ID NO: 20 oder SEQ ID NO: 29 bis 67 umfasst.

3. Vorläuferprotein nach einem der vorhergehenden Ansprüche, welches stabile Assoziate bildet, die durch 0,2 M NaOH innerhalb einer Stunde oder von 2 M Harnstoff oder 1 M Guanidiniumhydrochlorid innerhalb von 10 min bei Raumtemperatur nicht auflösbar sind.

4. Vorläuferprotein nach Anspruch 2 oder 3, worin das SA-Element eine Aminosäuresequenz, ausgewählt unter den Aminosäuresequenzen SEQ ID NO: 1 bis SEQ ID NO:5 und SEQ ID NO; 73, umfasst.

5. Vorläuferprotein nach einem der vorhergehenden Ansprüche, wobei wenigstens ein Aux-Peptid außerdem ein Schutzpeptid (SU)-Element umfasst, das einen erhöhten Anteil an negativ geladenen Aminosäureresten aufweist.

6. Vorläuferprotein nach Anspruch 5, wobei das SU-Element im Vorläuferprotein zur Ausbildung einer amphiphilen Helixstruktur befähigt ist.

7. Vorläuferprotein nach Anspruch 6, wobei das SU-Element ein amphiphiles Peptid ist, umfassend einen Sequenzabschnitt von wenigstens sieben peptidisch verknüpften Aminosäuren, die zur Ausbildung einer amphiphilen alpha-Helix befähigt sind, wobei die Helix in ihrer vertikalen Projektion eine Trennung der Aminosäurereste in eine hydrophobe und eine hydrophile Helixhälfte aufweist, die hydrophobe Helixhälfte mindestens 3 (in der vertikalen Projektion) benachbarte gleiche oder verschiedene hydrophobe Aminosäurereste aufweist und die hydrophile Helixhälfte mindestens 3 (in der vertikalen Projektion) benachbarte gleiche oder verschiedene hydrophile Aminosäurereste aufweist.

8. Vorläuferprotein nach Anspruch 5, 6 oder 7, wobei der Anteil an geladenen Aminosäureresten des SU-Elements so gewählt ist, dass die Gesamtnettoladung des Vorläuferproteins bei pH=7 größer als -10 und kleiner als +10.

9. Vorläuferprotein nach einem der Ansprüche 5 bis 8, wobei das SU-Element eine Aminosäuresequenz, ausgewählt unter den Aminosäuresequenzen SEQ ID NO: 16 bis SEQ ID NO:19 und SEQ ID NO: 68 umfasst.

10. Vorläuferprotein nach einem der Ansprüche 1 und 3 bis 9, wobei das Pep-Element eine kationische antimikrobielle Peptidsequenz umfasst.

11. Vorläuferprotein nach Anspruch 10, wobei das Pep-Element eine Aminosäuresequenz, ausgewählt unter den kationischen Aminosäuresequenzen SEQ ID NO: 6 bis SEQ ID NO:15, SEQ ID NO: 23, SEQ ID NO: 26 und SEQ ID NO: 69 bis SEQ ID NO: 72 umfasst.

12. Vorläuferprotein nach einem der Ansprüche 1, 3 oder 4, wobei das Pep-Element eine Aminosäuresequenz, ausgewählt unter den Aminosäuresequenzen SEQ ID NO: 20 oder SEQ ID NO: 29 bis 67 umfasst.

13. Vorläuferprotein nach einem der vorhergehenden Ansprüche wobei die Aux-Elemente unabhängig voneinander eine der folgenden Bedeutungen besitzen:
SA,
SA-SU,
SU-SA,
SA-SU-SA,
SU-SA-SU,
worin die Elemente SA und SU miteinander peptidisch verknüpft sind und die Aux-Elemente terminal mit wenigstens einem Pep-Element peptidisch verknüpft sind, und diese peptidische Verknüpfung zu den Pep-Elementen chemisch oder enzymatisch spezifisch spaltbar ist.

14. Nukleinsäuresequenz kodierend für wenigstens ein Vorläuferprotein nach einem der vorhergehenden Ansprüche.

15. Nukleinsäuresequenz nach Anspruch 14, umfassend wenigstens eine kodierende Sequenz gemäß SEQ ID NO: 21, 24, 27, 74, und 76.

16. Expressionskassette umfassend wenigstens eine Nukleinsäuresequenz nach Anspruch 14 oder 15, operativ verknüpft mit wenigstens einer regulatorischen Nukleinsäuresequenz.

17. Rekombinanter Vektor zur Transformation eines eukaryontischen oder prokaryontischen Wirts, umfassend eine Nukleinsäuresequenz gemäß einem der Ansprüche 14 oder 15, oder eine Expressionskassette gemäß Anspruch 16.

18. Verfahren zur Herstellung eines gewünschten Peptids (Pep), wobei man
a) ein Vorläuferprotein herstellt,
umfassend eine spaltbare repetitive Abfolge von gewünschten Peptid (Pep)-Elementen und Hilfspeptid (Aux)-Elementen, der allgemeinen Formel
(Pep-Aux)ₓ oder
(Aux-Pep)ₓ
worin x >1 ist, wobei
die Aux-Elemente gleich oder verschieden sind und Aminosäuresequenzelemente umfassen, die dem Vorläuferprotein selbstassemblierende Eigenschaften verleihen, wobei das Aux-Element ein selbstassemblierendes Peptid ((SA)-Element) umfasst
wobei das SA-Element wenigstens eines der folgenden Sequenzmotive enthält:
Aₙ (Motiv 1)
(GA)ₘ (Motiv 2)
Vₙ (Motiv 3)
(VA)ₘ (Motiv 4)
(VVAA)ₒ (Motiv 5)
worin A für Alanin, G für Glycin, V für Valin, n für einen ganzzahligen Wert von 8 bis 12, m für einen ganzzahligen Wert von 4 bis 10, und o für einen ganzzahligen Wert von 2 bis 6 steht; und
die Pep-Elemente gleich oder verschieden sind und die Aminosäuresequenz gleicher oder verschiedener Peptidmoleküle mit einer Sequenzlänge von 5-70 Aminosäureresten umfassen, und die Pep-Elemente von Spaltsequenzen flankiert sind, die eine spezifische Herausspaltung der Pep-Elemente aus dem Vorläuferprotein ermöglichen, wobei die Elemente Pep und Aux miteinander peptidisch verknüpft sind und die peptidische Verknüpfung chemisch spezifisch spaltbar ist, und
b) die Pep-Peptide aus dem Vorläuferprotein abspaltet.

19. Verfahren nach Anspruch 18, wobei man das Vorläuferprotein in einem rekombinanten Mikroorganismus produziert, der wenigstens einen Vektor nach Anspruch 17 trägt.

20. Verfahren nach Anspruch 18 oder 19, wobei das Vorläuferprotein stabile Assoziate bildet, die durch 0,2 M NaOH innerhalb einer Stunde oder von 2 M Harnstoff oder 1 M Guanidiumhydrochlorid innerhalb von 10 min bei Raumtemperatur nicht auflösbar sind.

21. Verfahren nach einem der Ansprüche 18 bis 20, wobei das Vorläuferprotein wie in einem der Ansprüche 4 bis 13 definiert ist, oder von einer Nukleinsäuresequenz nach einem der Ansprüche 14 und 15 kodiert wird.

## Claims

1. A precursor protein comprising a cleavable repetitive sequence of desired peptide (Pep) elements and auxiliary peptide (Aux) elements of the general formula
(Pep-Aux)ₓ or
(Aux-Pep)ₓ
where x >1, wherein
the Aux elements are identical or different and comprise amino acid sequence elements which impart to the precursor protein self-assembling properties, wherein the Aux element comprises a self-assembling peptide ((SA) element)
wherein the SA element comprises at least one of the following sequence motifs:
Aₙ (motif 1)
(GA)ₘ (motif 2)
Vₙ (motif 3)
(VA)ₘ (motif 4)
(VVAA)ₒ (motif 5)
where A is alanine, G is glycine, V is valine, n is an integer from 8 to 12, m is an integer from 4 to 10, and o is an integer from 2 to 6; and
the Pep elements are identical or different and comprise the amino acid sequence of identical or different peptide molecules having a sequence length of 5-70 amino acid residues, and the Pep elements are flanked by cleavage sequences which enable the Pep elements to be specifically cleaved out of the precursor protein, wherein
the elements Pep and Aux are peptidically linked to one another and the peptidic linkage is chemically cleavable in a specific manner, wherein the cleavage sequences comprise a sequence motif selected from among NG and DP.

2. A precursor protein comprising a cleavable repetitive sequence of desired peptide (Pep) elements and auxiliary peptide (Aux) elements of the general formula
(Pep-Aux)ₓ or
(Aux-Pep)ₓ
where x >1, wherein
the Aux elements are identical or different and comprise amino acid sequence elements which impart to the precursor protein self-assembling properties, wherein the Aux element comprises a self-assembling peptide ((SA) element)
wherein the SA element comprises at least one of the following sequence motifs:
Aₙ (motif 1)
(GA)ₘ (motif 2)
Vₙ (motif 3)
(VA)ₘ (motif 4)
(VVAA)ₒ (motif 5)
where A is alanine, G is glycine, V is valine, n is an integer from 8 to 12, m is an integer from 4 to 10, and o is an integer from 2 to 6; and
the Pep elements are identical or different and comprise the amino acid sequence of identical or different peptide molecules having a sequence length of 5-70 amino acid residues, and the Pep elements are flanked by cleavage sequences which enable the Pep elements to be specifically cleaved out of the precursor protein, wherein
the elements Pep and Aux are peptidically linked to one another and the peptidic linkage is chemically or enzymatically cleavable in a specific manner, wherein
a) the Pep element comprises an amino acid sequence selected from among the cationic amino acid sequences SEQ ID NO: 6 to SEQ ID NO:15, SEQ ID NO: 23, SEQ ID NO: 26 and SEQ ID NO: 69 to SEQ ID NO: 72, or
b) the Pep element comprises an amino acid sequence selected from among the amino acid sequences SEQ ID NO: 20 or SEQ ID NO: 29 to 67.

3. The precursor protein according to either of the preceding claims, which forms stable associates which cannot be dissolved at room temperature by 0.2 M NaOH inside one hour or by 2 M urea or 1 M guanidinium hydrochloride inside 10 min.

4. The precursor protein according to claim 2 or 3, wherein the SA element comprises an amino acid sequence selected from among the amino acid sequences SEQ ID NO: 1 to SEQ ID NO:5 and SEQ ID NO:73.

5. The precursor protein according to any of the preceding claims, wherein at least one Aux peptide additionally comprises a protective peptide (SU) element which
has an increased proportion of negatively charged amino acid residues.

6. The precursor protein according to claim 5, wherein the SU element in the precursor protein is capable of forming an amphiphilic helical structure.

7. The precursor protein according to claim 6, wherein the SU element is an amphiphilic peptide comprising a sequence segment of at least seven peptidically linked amino acids capable of forming an amphiphilic alpha-helix, wherein the amino acid residues of said helix in its vertical projection are separated into a hydrophobic half and a hydrophilic half of the helix, the hydrophobic half of the helix having at least 3 adjacent (in the vertical projection) identical or different hydrophobic amino acid residues, and the hydrophilic half of the helix having at least 3 adjacent (in the vertical projection) identical or different hydrophilic amino acid residues.

8. The precursor protein according to claim 5, 6 or 7, wherein the proportion of charged amino acid residues of the SU element is chosen such that the overall net charge of the precursor protein at pH=7 is greater than -10 and less than +10.

9. The precursor protein according to any of claims 5 to 8, wherein the SU element comprises an amino acid sequence selected from among the amino acid sequences SEQ ID NO: 16 to SEQ ID NO:19 and SEQ ID NO: 68.

10. The precursor protein according to any of claims 1 and 3 to 9, wherein the Pep element comprises a cationic antimicrobial peptide sequence.

11. The precursor protein according to claim 10, wherein the Pep element comprises an amino acid sequence selected from among the cationic amino acid sequences SEQ ID NO: 6 to SEQ ID NO:15, SEQ ID NO: 23, SEQ ID NO: 26 and SEQ ID NO: 69 to SEQ ID NO: 72.

12. The precursor protein according to any of claims 1, 3 and 4, wherein the Pep element comprises an amino acid sequence selected from among the amino acid sequences SEQ ID NO: 20 or SEQ ID NO: 29 to 67.

13. The precursor protein according to any of the preceding claims, wherein the Aux elements independently of one another have one of the following meanings:
SA,
SA-SU,
SU-SA,
SA-SU-SA,
SU-SA-SU,
wherein the elements SA and SU are peptidically linked to one another, and the Aux elements are peptidically linked terminally to at least one Pep element, and this peptidic linkage to the Pep elements is chemically or enzymatically clearable in a specific manner.

14. A nucleic acid sequence coding for at least one precursor protein according to any of the preceding claims.

15. The nucleic acid sequence according to claim 14, comprising at least one coding sequence of sequence SEQ ID NO: 21, 24, 27, 74 and 76.

16. An expression cassette comprising at least one nucleic acid sequence according to claim 14 or 15, operatively linked to at least one regulatory nucleic acid sequence.

17. A recombinant vector for transforming a eukaryotic or prokaryotic host, comprising a nucleic acid sequence according to either of claims 14 and 15, or an expression cassette according to claim 16.

18. A method of producing a desired peptide (Pep), which comprises
a) producing a precursor protein,
comprising a cleavable repetitive sequence of desired peptide (Pep) elements and auxiliary peptide (Aux) elements of the general formula
(Pep-Aux)ₓ or
(Aux-Pep)ₓ
where x >1, wherein
the Aux elements are identical or different and comprise amino acid sequence elements which impart to the precursor protein self-assembling properties, wherein the Aux element comprises a self-assembling peptide ((SA) element)
wherein the SA element comprises at least one of the following sequence motifs:
Aₙ (motif 1)
(GA)ₘ (motif 2)
Vₙ (motif 3)
(VA)ₘ (motif 4)
(VVAA)ₒ (motif 5)
where A is alanine, G is glycine, V is valine, n is an integer from 8 to 12, m is an integer from 4 to 10, and o is an integer from 2 to 6; and
the Pep elements are identical or different and comprise the amino acid sequence of identical or different peptide molecules having a sequence length of 5-70 amino acid residues, and the Pep elements are flanked by cleavage sequences which enable the Pep elements to be specifically cleaved out of the precursor protein, wherein the elements Pep and Aux are peptidically linked to one another and the peptidic linkage is chemically cleavable in a specific manner, and
b) removing the Pep peptides from the precursor protein.

19. The method according to claim 18, wherein the precursor protein is produced in a recombinant microorganism carrying at least one vector according to claim 17.

20. The method according to claim 18 or 19, wherein the precursor protein forms stable associates which cannot be dissolved at room temperature by 0.2 M NaOH inside one hour or by 2 M urea or 1 M guanidinium hydrochloride inside 10 min.

21. The method according to any of claims 18 to 20, wherein the precursor protein is as defined in any of claims 4 to 13, or is encoded by a nucleic acid sequence according to either of claims 14 and 15.

## Revendications

1. Protéine précurseur, comprenant une séquence répétitive clivable d'éléments peptide (Pep) et d'éléments peptide auxiliaire (Aux) souhaités, de la formule générale :
(Pep-Aux)ₓ ou
(Aux-Pep)ₓ
avec x > 1,
les éléments Aux étant identiques ou différents, et comprenant des éléments séquence d'acides aminés qui confèrent à la protéine précurseur des propriétés d'auto-assemblage, l'élément Aux comprenant un peptide à auto-assemblage (élément (SA)),
l'élément SA contenant au moins un des motifs de séquence suivants :
Aₙ (motif 1)
(GA)ₘ (motif 2)
Vₙ (motif 3)
(VA)ₘ (motif 4)
(VVAA)ₒ (motif 5)
A représentant l'alanine, G représentant la glycine, V représentant la valine, n représentant une valeur de nombre entier de 8 à 12, m représentant une valeur de nombre entier de 4 à 10, et o représentant une valeur de nombre entier de 2 à 6 ; et
les éléments Pep étant identiques ou différents, et comprenant la séquence d'acides aminés de molécules peptidiques identiques ou différentes d'une longueur de séquence de 5 à 70 radicaux acides aminés, et les éléments Pep étant flanqués par des séquences de clivage, qui permettent un clivage spécifique des éléments Pep à partir de la protéine précurseur, les éléments Pep et Aux étant reliés peptidiquement les uns avec les autres, et la liaison peptidique étant clivable chimiquement d'une manière spécifique, les séquences de clivage comprenant un motif de séquence choisi parmi NG et DP.

2. Protéine précurseur, comprenant une séquence répétitive clivable d'éléments peptide (Pep) et d'éléments peptide auxiliaire (Aux) souhaités, de la formule générale :
(Pep-Aux)ₓ ou
(Aux-Pep)ₓ
avec x > 1,
les éléments Aux étant identiques ou différents, et comprenant des éléments séquence d'acides aminés qui confèrent à la protéine précurseur des propriétés d'auto-assemblage, l'élément Aux comprenant un peptide à auto-assemblage (élément (SA)),
l'élément SA contenant au moins un des motifs de séquence suivants :
Aₙ (motif 1)
(GA)ₘ (motif 2)
Vₙ (motif 3)
(VA)ₘ (motif 4)
(VVAA)ₒ (motif 5)
A représentant l'alanine, G représentant la glycine, V représentant la valine, n représentant une valeur de nombre entier de 8 à 12, m représentant une valeur de nombre entier de 4 à 10, et o représentant une valeur de nombre entier de 2 à 6 ; et
les éléments Pep étant identiques ou différents, et comprenant la séquence d'acides aminés de molécules peptidiques identiques ou différentes d'une longueur de séquence de 5 à 70 radicaux acides aminés, et les éléments Pep étant flanqués par des séquences de clivage, qui permettent un clivage spécifique des éléments Pep à partir de la protéine précurseur,
les éléments Pep et Aux étant reliés peptidiquement les uns avec les autres, et la liaison peptidique étant clivable chimiquement ou enzymatiquement d'une manière spécifique,
a) l'élément Pep comprenant une séquence d'acides aminés choisie parmi les séquences d'acides aminés cationiques SEQ ID NO : 6 à SEQ ID NO : 15, SEQ ID NO : 23, SEQ ID NO : 26 et SEQ ID NO : 69 à SEQ ID NO : 72, ou
b) l'élément Pep comprenant une séquence d'acides aminés choisie parmi les séquences d'acides aminés SEQ ID NO : 20 ou SEQ ID NO : 29 à 67.

3. Protéine précurseur selon l'une quelconque des revendications précédentes, qui forme des produits d'association stables, qui ne sont pas dissolvables par du NaOH 0,2 M en une heure ou par de l'urée 2 M ou du chlorhydrate de guanidinium 1 M en 10 minutes à température ambiante.

4. Protéine précurseur selon la revendication 2 ou 3, dans laquelle l'élément SA comprend une séquence d'acides aminés choisie parmi les séquences d'acides aminés SEQ ID NO : 1 à SEQ ID NO : 5 et SEQ ID NO : 73.

5. Protéine précurseur selon l'une quelconque des revendications précédentes, dans laquelle au moins un peptide Aux comprend en outre un élément peptide protecteur (SU), qui comprend une proportion élevée de radicaux acides aminés chargés négativement.

6. Protéine précurseur selon la revendication 5, dans laquelle l'élément SU dans la protéine précurseur est apte à la formation d'une structure d'hélice amphiphile.

7. Protéine précurseur selon la revendication 6, dans laquelle l'élément SU est un peptide amphiphile, comprenant une section de séquence d'au moins sept acides aminés reliés peptidiquement, qui sont aptes à la formation d'une hélice alpha amphiphile, l'hélice comprenant dans sa projection verticale une séparation des radicaux acides aminés en une moitié d'hélice hydrophobe et une moitié d'hélice hydrophile, la moitié d'hélice hydrophobe comprenant au moins 3 (dans la projection verticale) radicaux acides aminés hydrophobes identiques ou différents voisins, et la moitié d'hélice hydrophile comprenant au moins 3 (dans la projection verticale) radicaux acides aminés hydrophiles identiques ou différents voisins.

8. Protéine précurseur selon la revendication 5, 6 ou 7, dans laquelle la proportion de radicaux acides aminés chargés de l'élément SU est choisie de telle sorte que la charge nette totale de la protéine précurseur à pH = 7 soit supérieure à -10 et inférieure à +10.

9. Protéine précurseur selon l'une quelconque des revendications 5 à 8, dans laquelle l'élément SU comprend une séquence d'acides aminés choisie parmi les séquences d'acides aminés SEQ ID NO : 16 à SEQ ID NO : 19 et SEQ ID NO : 68.

10. Protéine précurseur selon l'une quelconque des revendications 1 et 3 à 9, dans laquelle l'élément Pep comprend une séquence peptidique antimicrobienne cationique.

11. Protéine précurseur selon la revendication 10, dans laquelle l'élément Pep comprend une séquence d'acides aminés choisie parmi les séquences d'acides aminés cationiques SEQ ID NO : 6 à SEQ ID NO : 15, SEQ ID NO : 23, SEQ ID NO : 26 et SEQ ID NO : 69 à SEQ ID NO : 72.

12. Protéine précurseur selon l'une quelconque des revendications 1, 3 ou 4, dans laquelle l'élément Pep comprend une séquence d'acide aminés choisie parmi les séquences d'acides aminés SEQ ID NO : 20 ou SEQ ID NO : 29 à 67.

13. Protéine précurseur selon l'une quelconque des revendications précédentes, dans laquelle les éléments Aux présentent indépendamment les uns des autres une des significations suivantes :
SA,
SA-SU,
SU-SA,
SA-SU-SA,
SU-SA-SU,
les éléments SA et SU étant reliés peptidiquement l'un avec l'autre, et les éléments Aux étant reliés peptidiquement en position terminale avec au moins un élément Pep, et cette liaison peptidique avec les éléments Pep étant clivable chimiquement ou enzymatiquement d'une manière spécifique.

14. Séquence d'acides nucléiques codant pour au moins une protéine précurseur selon l'une quelconque des revendications précédentes.

15. Séquence d'acides nucléiques selon la revendication 14, comprenant au moins une séquence codante selon SEQ ID NO : 21, 24, 27, 74 et 76.

16. Cassette d'expression comprenant au moins une séquence d'acides nucléiques selon la revendication 14 ou 15, reliée opérationnellement avec au moins une séquence d'acides nucléiques de régulation.

17. Vecteur recombinant pour la transformation d'un hôte eucaryote ou procaryote, comprenant une séquence d'acides nucléiques selon l'une quelconque des revendications 14 ou 15, ou une cassette d'expression selon la revendication 16.

18. Procédé de fabrication d'un peptide souhaité (Pep), selon lequel
a) une protéine précurseur est fabriquée,
comprenant une séquence répétitive clivable d'éléments peptide (Pep) et d'éléments peptide auxiliaire (Aux) souhaités, de la formule générale :
(Pep-Aux)ₓ ou
(Aux-Pep)ₓ
avec x > 1,
les éléments Aux étant identiques ou différents, et comprenant des éléments séquence d'acides aminés qui confèrent à la protéine précurseur des propriétés d'auto-assemblage, l'élément Aux comprenant un peptide à auto-assemblage (élément (SA)),
l'élément SA contenant au moins un des motifs de séquence suivants :
Aₙ (motif 1)
(GA)ₘ (motif 2)
Vₙ (motif 3)
(VA)ₘ (motif 4)
(VVAA)ₒ (motif 5)
A représentant l'alanine, G représentant la glycine, V représentant la valine, n représentant une valeur de nombre entier de 8 à 12, m représentant une valeur de nombre entier de 4 à 10, et o représentant une valeur de nombre entier de 2 à 6 ; et
les éléments Pep étant identiques ou différents, et comprenant la séquence d'acides aminés de molécules peptidiques identiques ou différentes d'une longueur de séquence de 5 à 70 radicaux acides aminés, et les éléments Pep étant flanqués par des séquences de clivage, qui permettent un clivage spécifique des éléments Pep à partir de la protéine précurseur, les éléments Pep et Aux étant reliés peptidiquement les uns avec les autres, et la liaison peptidique étant clivable chimiquement d'une manière spécifique, et
b) les peptides Pep sont clivés de la protéine précurseur.

19. Procédé selon la revendication 18, dans lequel la protéine précurseur est produite dans un microorganisme recombinant qui porte au moins un vecteur selon la revendication 17.

20. Procédé selon la revendication 18 ou 19, dans lequel la protéine précurseur forme des produits d'association stables, qui ne sont pas dissolvables par du NaOH 0,2 M en une heure ou par de l'urée 2 M ou du chlorhydrate de guanidinium 1 M en 10 minutes à température ambiante.

21. Procédé selon l'une quelconque des revendications 18 à 20, dans lequel la protéine précurseur est telle que définie dans l'une quelconque des revendications 4 à 13, ou est codée par une séquence d'acides nucléiques selon l'une quelconque des revendications 14 et 15.
